# EUROPEAN PATENT SPECIFICATION

(11) **EP 3 579 897 B1**
(45) Date of publication and mention of the grant of the patent: **11.11.2026**
(21) Application number: 18764628.6
(22) Date of filing: 12.03.2018
(51) Int. Cl.: A61K 31/727, B01D 15/36, A61P 7/02

(54) **SHORT-ACTING HEPARIN-BASED ANTICOAGULANT COMPOUNDS AND USE THEREOF**
KURZFRISTIG WIRKENDE ANTIKOAGULIERENDE VERBINDUNGEN AUF HEPARINBASIS UND IHRE VERWENDUNG
COMPOSÉS ANTICOAGULANTS À BASE D'HÉPARINE À COURTE DURÉE D'ACTION ET LEUR UTILISATION

(30) Priority: 10.03.2017 US 201762469643 P
(43) Date of publication of application: 18.12.2019
(73) Proprietor: The University of North Carolina at Chapel Hill, Chapel Hill, NC 27514 (US)
(72) Inventor: LIU, Jian, Chapel Hill, NC 27514 (US); WANG, Zhangjie, Changchun Jilin Province 130000 (CN); HSIEH, Po-Hung, Pasadena, CA 91106 (US); XU, Yongmei, Durham, NC 27707 (US)
(74) Representative: Isarpatent
(86) International application number: PCT/US2018/021986
(87) International publication number: WO 2018/165656

(56) References cited:
- US-A1- 2012 322 114
- US-A1- 2013 296 540
- US-A1- 2016 122 446
- DING XU ET AL: "Engineering sulfotransferases to modify heparan sulfate", NATURE CHEMICAL BIOLOGY, vol. 4, no. 3, 27 January 2008 (2008-01-27), New York, pages 200 - 202, XP055752842, ISSN: 1552-4450, DOI: 10.1038/nchembio.66
- Y. XU ET AL: "Chemoenzymatic Synthesis of Homogeneous Ultralow Molecular Weight Heparins", SCIENCE, vol. 334, no. 6055, 27 October 2011 (2011-10-27), pages 498 - 501, XP055126707, ISSN: 0036-8075, DOI: 10.1126/science.1207478
- DAS SANJOY K ET AL: "Synthesis of conformationally locked L-iduronic acid derivatives: direct evidence for a critical role of the skew-boat 2S0 conformer in the activation of antithrombin by heparin", CHEMISTRY - A EUROPEAN JOURNAL, WILEY - V C H VERLAG GMBH & CO. KGAA, DE, vol. 7, no. 22, 1 January 2001 (2001-01-01), pages 4821 - 4834, XP002197423, ISSN: 0947-6539, DOI: 10.1002/1521-3765(20011119)7:22<4821::AID-CHEM4821>3.3.CO;2-E
- COPELAND ET AL.: "Using a 3-O-Sulfated Heparin Octasaccharide To Inhibit the Entry of Herpes Simplex Virus Type 1", BIOCHEMISTRY, vol. 47, no. 21, 6 May 2008 (2008-05-06), pages 5774 - 5783, XP002740123

## Description

### TECHNICAL FIELD

The subject matter disclosed herein relates to pharmaceutical compositions comprising synthetic heparin compounds. Furthermore, the subject matter disclosed herein relates to synthetic heparin analogues for use in a method of treating a subject in need of anticoagulant activity.

### BACKGROUND

Heparan sulfate (HS) is a ubiquitous component of the cell surface and extracellular matrix. It regulates a wide range of physiologic and pathophysiologic functions, including embryonic development and blood coagulation, and can facilitate viral infection (Esko and Selleck (2002) Annu. Rev. Biochem. 71, 435-471; Liu and Thorp (2002) Med. Res. Rev. 22, 1-25). HS exerts its biological effects by interacting with the specific proteins involved in a given process (Capila and Lindhardt (2002) Angew. Chem. Int. Ed. 41, 390-412). HS is a highly charged polysaccharide comprising 1 → 4-linked glucosamine and glucuronic/iduronic acid units that contain both *N*- and *O-*sulfo groups. Unique saccharide sequences within HS can determine the specificity of the binding of HS to its target proteins (Linhardt (2003) J. Med. Chem.46, 2551-2564). Heparin, a specialized form of HS, is a commonly used anticoagulant drug. Thus, new methods for the synthesis of heparin compounds and HS attract considerable interest for those developing anticoagulant and other HS-related drugs having improved pharmacological effects.

Heparin has been successfully used as an anticoagulant drug for over 50 years (Mackman, 2008). It is currently marketed in three forms: unfractionated (UF) heparin (MW_{avg} ~14000 Da); a low molecular weight heparin (MW_{avg} ~6000 Da); and the synthetic ULMW heparin pentasaccharide ARIXTRA^{®} (MW 1508.3 Da). UF heparin is used in surgery and kidney dialysis due to its relatively short half-life and its safety for renal impaired patients (Hirsh et al., 2007).

D. Xu et al., Nature Chemical Biology (2008), 4, 200-202 disclose the engineering of sulfotransferases to modify heparan sulfate. R. Copeland et al., Biochemistry (2008), 47, 5774-5783 describe a 3-O-sulfated heparin octasaccharide for inhibiting the entry of herpes simplex virus type 1. US 2016/122446 A1 and US 2013/296540 A1 disclose methods and systems for synthesizing heparin compounds.

The synthesis of HS oligosaccharides and related anticoagulant compounds remains a challenge. Cost-effective methods and approaches for synthesizing new synthetic heparins are highly desirable.

### SUMMARY

Any references in the description to methods of treatment by therapy or surgery or in vivo diagnosis methods refer to the compounds, pharmaceutical compositions and medicaments of the present invention for use in those methods.

The present invention refers to a pharmaceutical composition comprising a synthetic heparin compound and to a synthetic heparin analogue for use in a method of treating a subject in need of anticoagulant therapy as set out in the appended claims.

The synthetic heparin analogues have an anticoagulant activity. In some embodiments such synthetic heparin analogues can have a binding affinity to antithrombin ranging from about 5 nM to about 30 nM. In some embodiments such synthetic heparin analogues can have anti-Xa activity ranging from about 10 ngml⁻¹ to about 40 ngml⁻¹ IC₅₀.

In some embodiments such synthetic heparin analogues can have a clearance rate about 50% to about 100% faster than that of other heparin compounds. In some embodiments such synthetic heparin analogues do not cause heparin-induced thrombocytopenia (HIT). In some embodiments such synthetic heparin analogues are reversible by andexanet alfa at a rate of 50% or more in the presence of 20ug/ml or less of andexanet alfa.

Described herein, but not claimed, are methods of synthesizing a synthetic heparin analogue, comprising providing a saccharide substrate, elongating the saccharide substrate to a saccharide of a desired or predetermined length, and performing at least one sulfation reaction using a 3-OST-3 isoform of a 3-O-sulfotransferase (3-OST) enzyme, whereby a synthetic heparin analogue is synthesized. In some aspects the saccharide substrate comprises at least one IdoA2S-GIcNS3S disaccharide unit. In some aspects the saccharide substrate comprises a IdoA2S-GIcNS3S±6S disaccharide unit, wherein the method further comprises a 6-O-sulfation step using a 6-O-sulfotransferase (6-OST), wherein a 3-*O*-sulfation by 3-OST-3 occurs prior to the 6-*O*-sulfation step. In some aspects the saccharide substrate comprises a GlcA-GlcNS3S6S disaccharide unit, wherein the method further comprises a 6-O-sulfation step using a 6-O-sulfotransferase (6-OST), wherein a 3-O-sulfation by 3-OST-1 occurs prior to the 6-*O*-sulfation step.

In some aspects the elongation step comprises employing a glycosyl transferase. In some embodiments the glycosyl transferase is selected from the group consisting of *N*-acetyl glucosaminyl transferase of *E*. *coli* K5 (KfiA) and/or heparosan synthase-2 (pmHS2) from *Pasteurella multocida.* In some embodiments the elongation step comprises employing one or more monosaccharides selected from the group consisting of: glucuronic acid (GlcUA), N-acetylated glucosamine (GIcNAc), and N-trifluoroacetyl glucosamine (GlcNTFA). In some aspects the method of synthesizing the synthetic heparin analogue has a yield of greater than about 20% to about 50%.

In some embodiments, the methods of treating a subject in need of anticoagulant therapy comprises providing a subject in need of anticoagulant therapy and administering to the subject a synthetic heparin analogue having anticoagulant activity. In some embodiments such methods further comprise monitoring the subject for heparin-induced thrombocytopenia, and administering to the subject an antidote to reverse the anticoagulant activity of the synthetic heparin analogue if the subject suffers from heparin-induced thrombocytopenia. In some embodiments the antidote to reverse the anticoagulant activity of the synthetic heparin analogue is andexanet alfa. In some embodiments the subject is a human subject.

In such methods of treating a subject the synthetic heparin analogue can have a clearance rate about 50% to about 100% faster than that of other heparin compounds. The synthetic heparin analogue can comprise an anticoagulant activity of less than about 10% at 4 hours post-administration. In such methods the subject can have an elevated risk of bleeding.

The synthetic heparin analogues comprise a structure selected from the group consisting of: 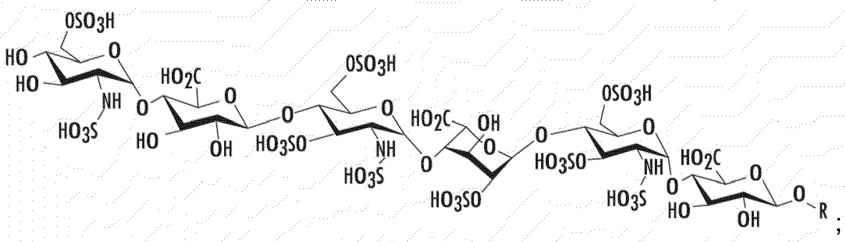 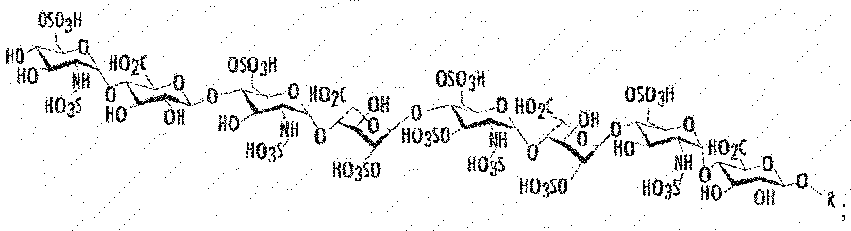 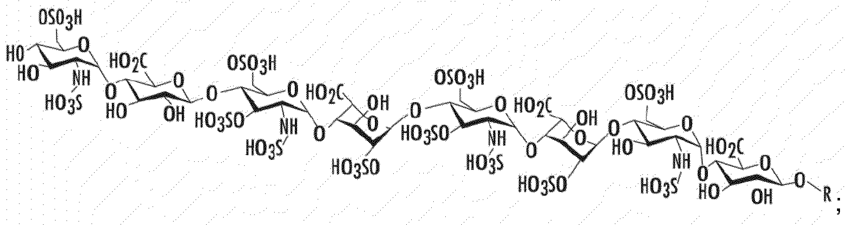 and 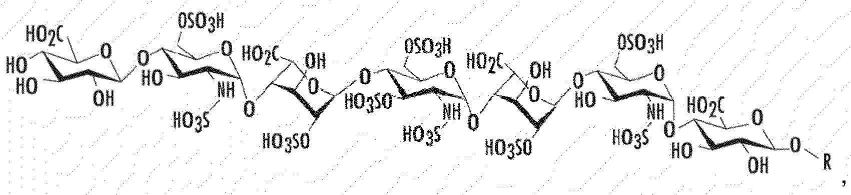 wherein R is selected from the group consisting of -H, alkyl (such as but not limited to -CH₃ or -CH₂CH₃), substituted alkyl, aryl, and substituted aryl (such as but not limited to a p-nitropheyl group).

Accordingly, it is an object of the presently disclosed subject matter to provide short-acting heparin-based anticoagulant compounds and methods, including a new form of synthetic heparin.

This and other objects are achieved in whole or in part by the presently disclosed subject matter. Further, an object of the presently disclosed subject matter having been stated above, other objects and advantages of the presently disclosed subject matter will become apparent to those skilled in the art after a study of the following description, Drawings and Examples.

### BRIEF DESCRIPTION OF THE DRAWINGS

The presently disclosed subject matter can be better understood by referring to the following figures. The components in the figures are not necessarily to scale, emphasis instead being placed upon illustrating the principles of the presently disclosed subject matter (often schematically). In the figures, like reference numerals designate corresponding parts throughout the different views. A further understanding of the presently disclosed subject matter can be obtained by reference to an embodiment set forth in the illustrations of the accompanying drawings. Although the illustrated embodiment is merely exemplary of systems for carrying out the presently disclosed subject matter, both the organization and method of operation of the presently disclosed subject matter, in general, together with further objectives and advantages thereof, may be more easily understood by reference to the drawings and the following description. The drawings are not intended to limit the scope of this presently disclosed subject matter, which is set forth in the claims as appended.

For a more complete understanding of the presently disclosed subject matter, reference is now made to the following drawings in which:

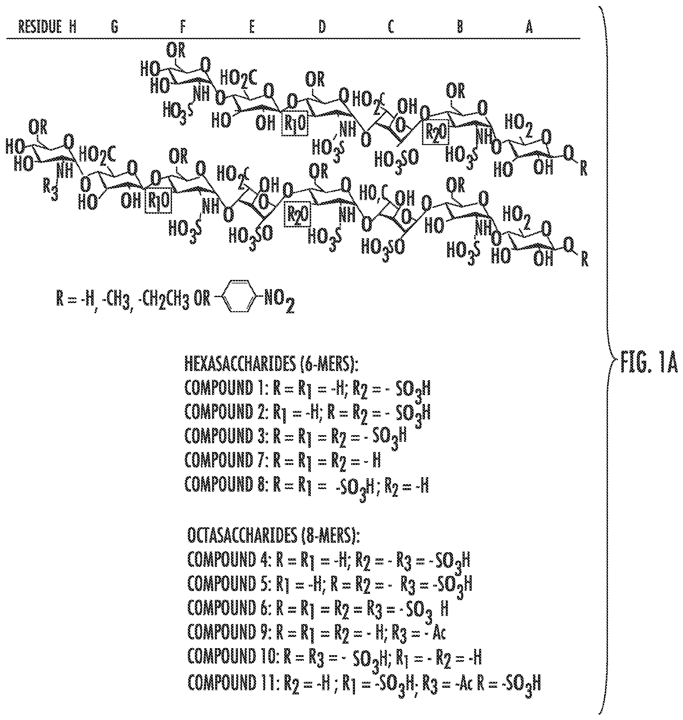

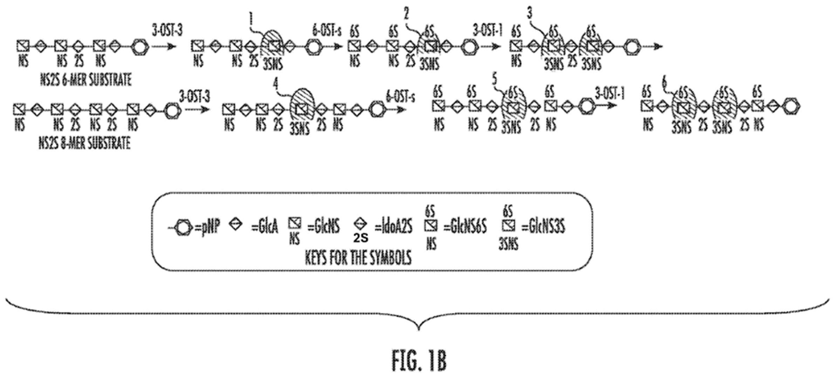

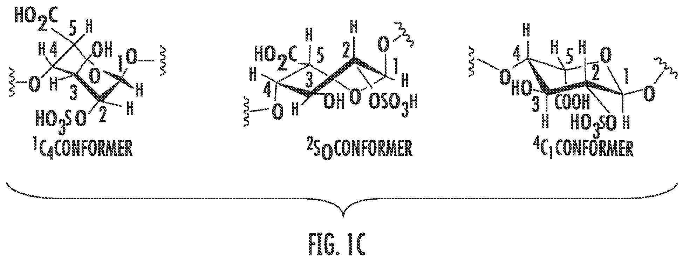

FIGS. 1A through 1C illustrate the synthesis of hexasaccharides and octasaccharides disclosed and tested herein, including FIG. 1A which a schematic illustration of hexasaccharides and octasaccharides disclosed herein, FIG. 1B which is a schematic illustration of an exemplary method of synthesiszing hexasaccharides and octasaccharides disclosed herein, and FIG. 1C illustrates chemical structures of pyranose rings of IdoA2S residues of hexasaccharides and octasaccharides disclosed herein;

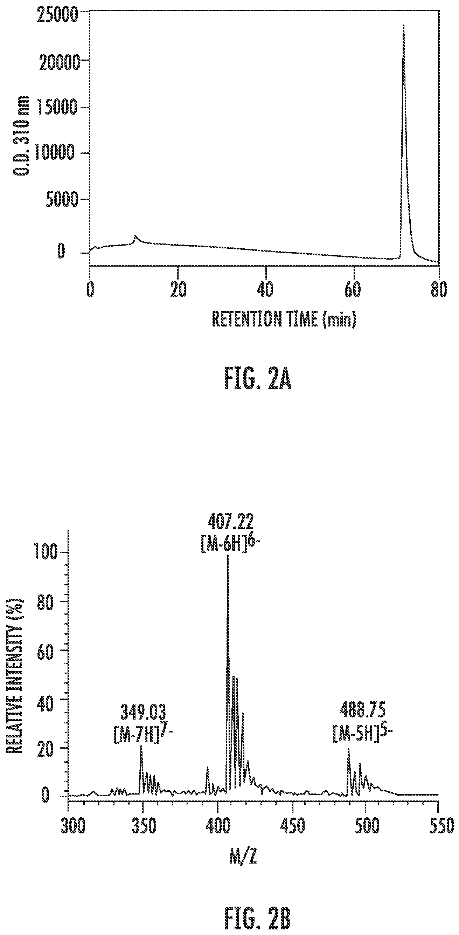

FIG. 2A is a graphical depiction of results of high resolution anion exchange HPLC, and FIG. 2B shows the results of electrospray ionization mass spectrometry (ESI-MS), both conducted on compound **6** to demonstrate the purity and structural analysis of the oligosaccharides disclosed herein;

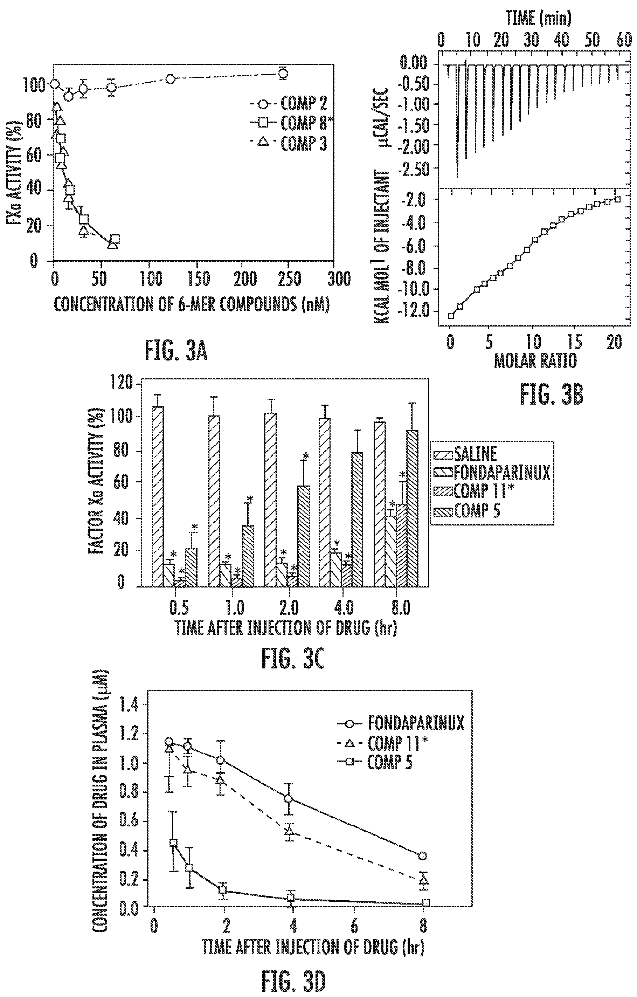

FIGS. 3A through 3D are graphical summaries of results illustrating the effects of the presence of the GIcNS3S6S-IdoA2S- disaccharide unit on anticoagulant activity;

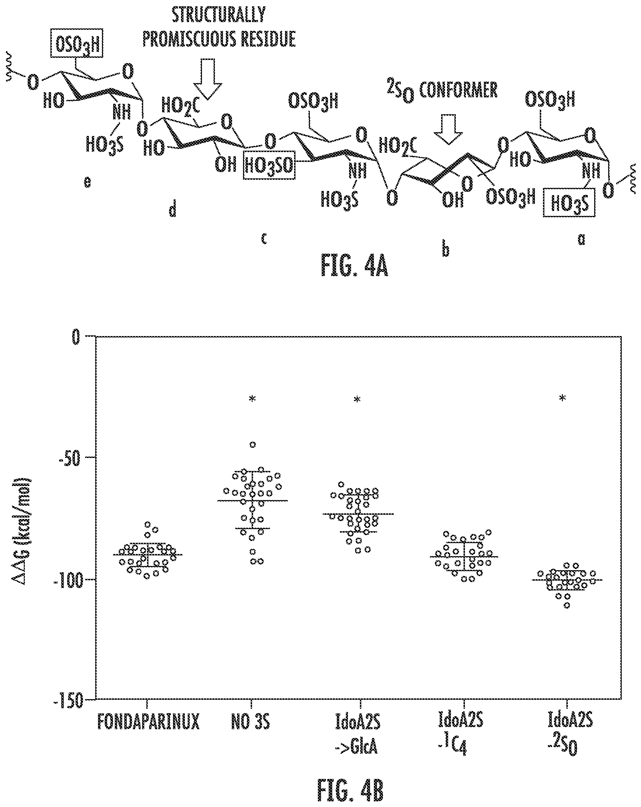

FIG. 4A schematic of the chemical structure of the AT-binding site with FIG. 4B graphically depicting results of molecular dynamics (MD) simulations of AT in complex;

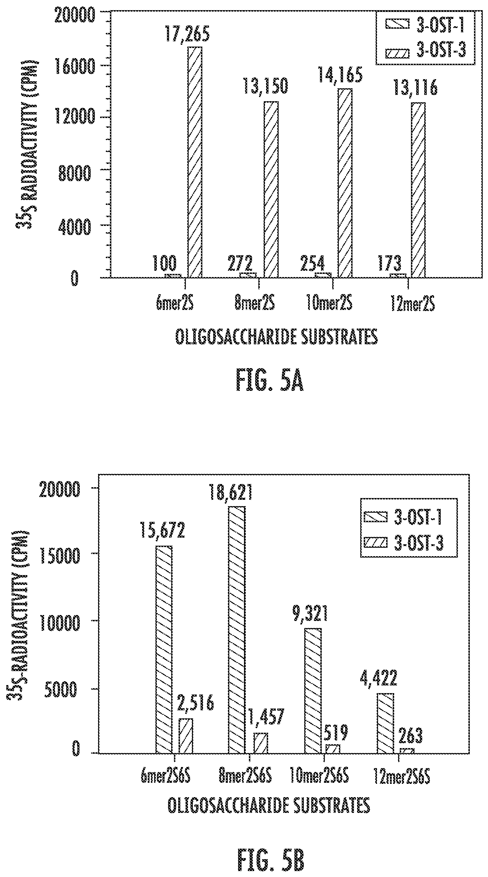

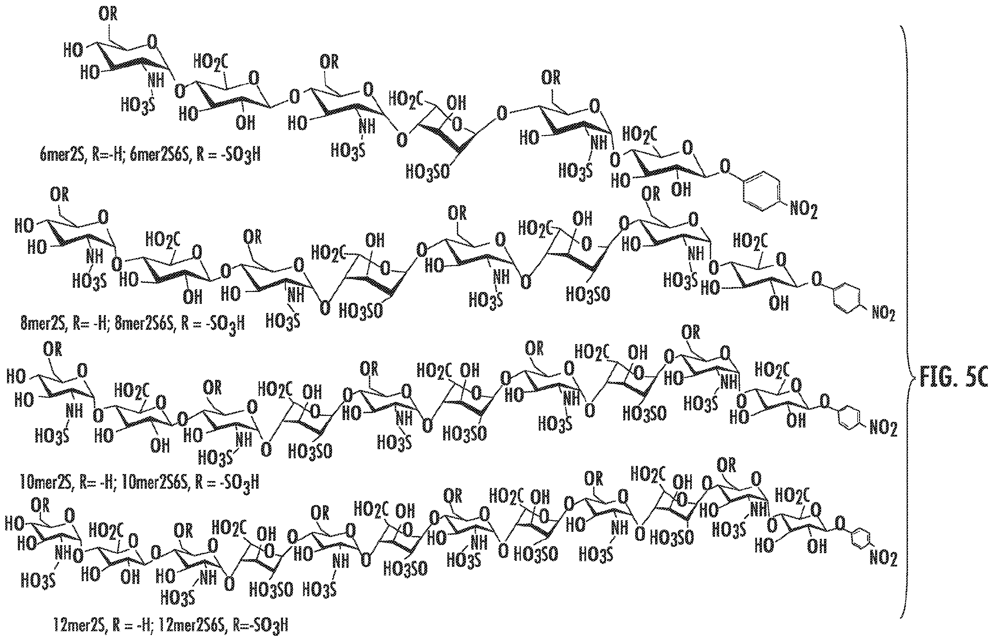

FIGS. 5A through 5C illustrate the substrate requirements for 3-OST-1 and 3-OST-3 using different sizes of structurally homogeneous oligosaccharides, where FIGS. 5A and 5B are bar graphs showing the substrate requirements for 3-OST-1 and 3-OST-3, where FIG. 5C illustrates the chemical structures of the different sized oligosaccharides;

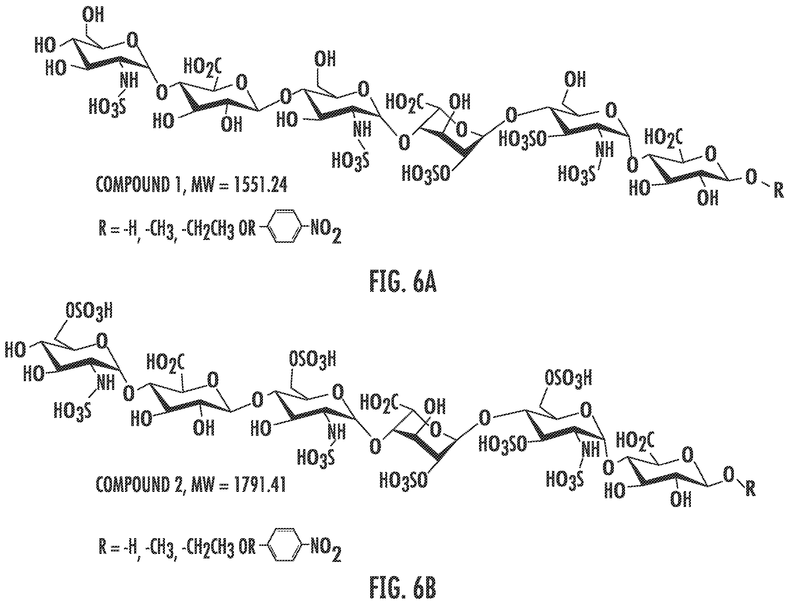

FIGS. 6A through 6F are schematics of the chemical structures of compounds 1, 2, 3, 4, 5 and 6, respectively;

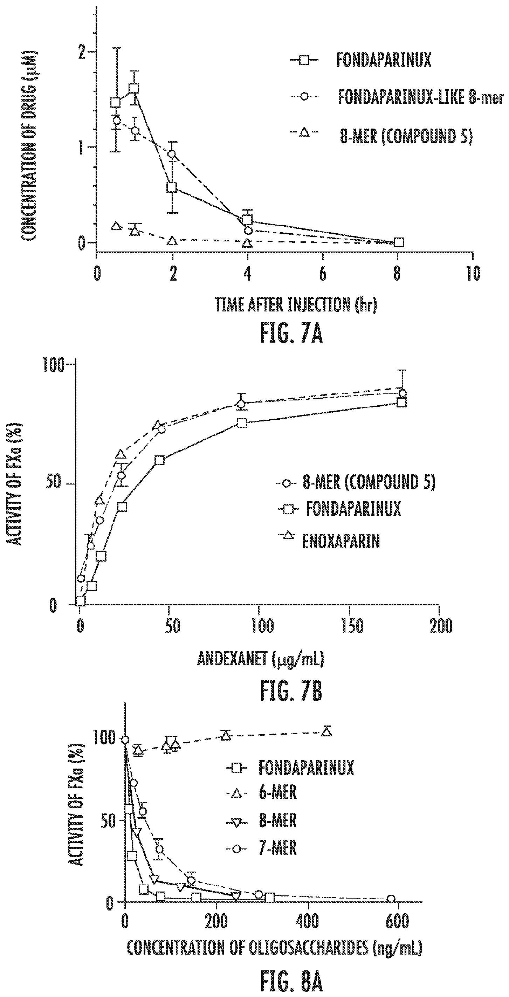

FIG. 7A is a graphical summary of data demonstrating that heparin compounds disclosed herein have faster clearance than fondaparinux in a C57BL/6J mouse model, and FIG. 7B is a graphical summary of data demonstrating that the anti-Fxa activity of heparin compounds disclosed herein is reversible by andexanet alfa (AndexXa^{®}, Portola Pharmaceuticals, South San Francisco, California, United States of America); and

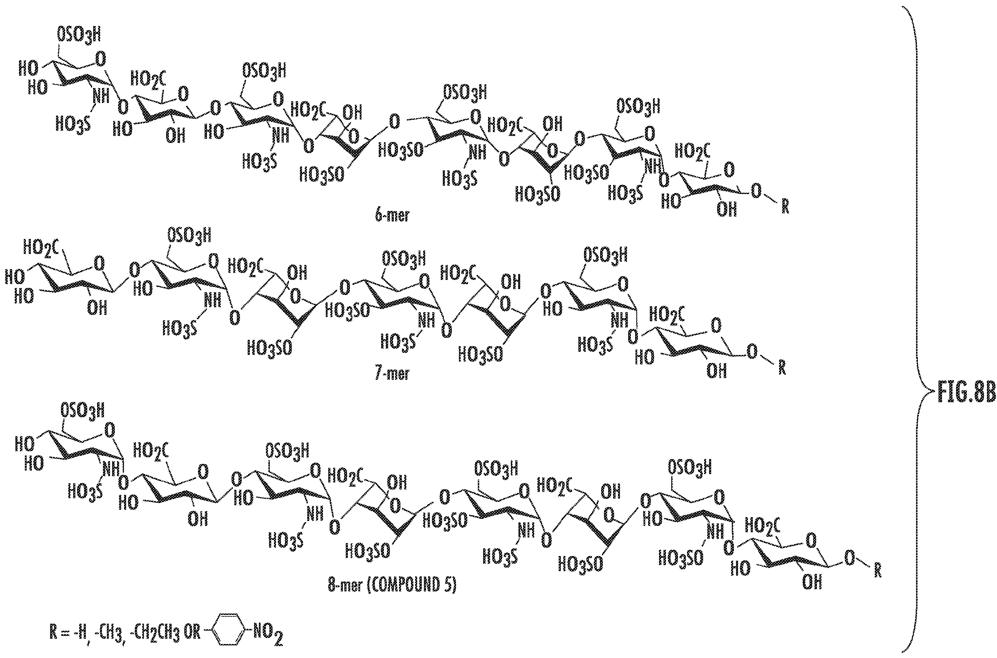

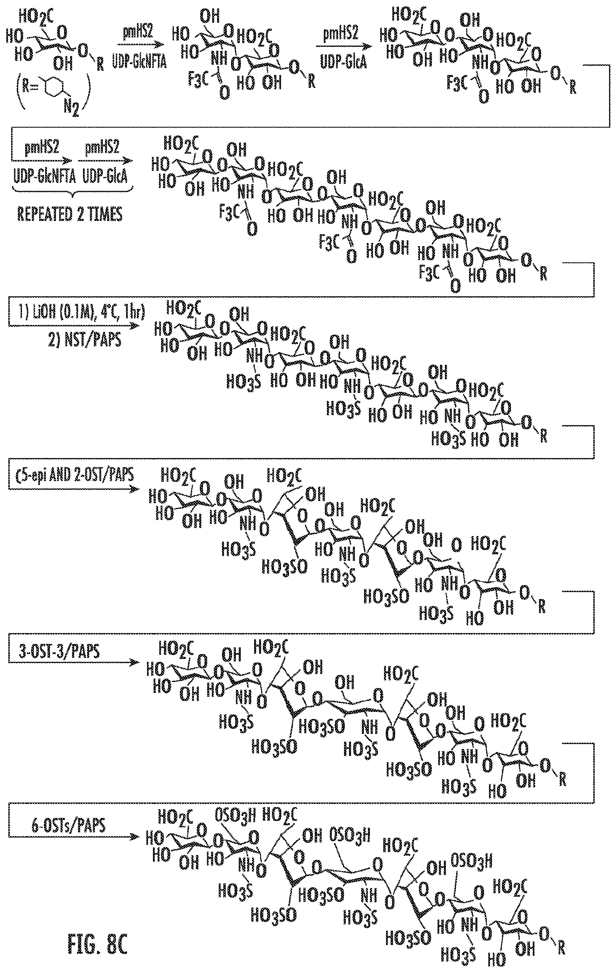

FIGS. 8A through 8C depict results of the analysis of a 7-mer HS compound disclosed herein, with FIG. 8A being a graphical summary of data showing the 7-mer has anti-Xa activity, FIG. 8B schematically comparing the chemical structural of the 7-mer to a 6-mer and 8-mer as disclosed herein, and FIG. 8C illustrating the synthetic pathway for synthesizing the 7-mer.

### DETAILED DESCRIPTION

The sulfation at the 3-OH position of glucosamine is at least one important modification in forming structural domains for heparan sulfate to enable its biological functions. Seven 3-*O*-sulfotransferase isoforms in the human genome are involved in the biosynthesis of 3-*O*-sulfated heparan sulfate. As a rare modification present in heparan sulfate, the availability of 3-*O*-sulfated oligosaccharides is very limited. Disclosed herein is a novel chemoenzymatic synthetic approach to synthesize six 3-*O*-sulfated oligosaccharides, including three hexasaccharides and three octasaccharides. The synthesis was achieved by rearranging the enzymatic modification sequence to accommodate the substrate specificity of 3-*O-*sulfotransferase 3. The impact of 3-*O*-sulfation on the conformation of the pyranose ring of 2-*O*-sulfated iduronic acid using NMR, and on the correlation between ring conformation and anticoagulant activity, was studied. From this a novel octasaccharide that interacts with antithrombin and displays anti-factor Xa activity was discovered. Interestingly, the octasaccharide displays a faster clearance rate than fondaparinux, an approved pentasaccharide drug, in a rat model, making this octasaccharide a potential short-acting anticoagulant drug candidate that could reduce bleeding risk. The disclosed 3-*O*-sulfated oligosaccharides also provide for new heparan sulfate-based therapeutics.

Heparan sulfate (HS) is a polysaccharide comprising disaccharide repeating units of a glucuronic acid (GlcA) or iduronic acid (IdoA) residue linked to a glucosamine (GlcN) residue, with each capable of being modified by sulfation. HS exhibits important physiological and pathological functions, including regulating embryonic development, inflammatory responses, blood coagulation and viral/bacterial infections¹⁻³. Most notably, heparin, a highly sulfated form of HS, is a widely used anticoagulant drug in clinics for the treatment of patients with thrombotic disorders⁴.

The functional selectivity of HS and heparin can be governed, at least in part, by the sulfation types and the location of GlcA and IdoA residues⁵. Sulfation is found at the 2-OH of IdoA (to a lesser extent GlcA) and the N-, 3-OH and 6-OH positions of GlcN residues. Moreover, the conformation of the IdoA and IdoA2S residues adopt both chair (*¹C4*) and skew boat (*²SO*) conformations⁶. The conformations of GlcA, GIcA2S and GIcN residues exist in the chair (*⁴C1*) conformation^{7,8}. The conformational flexibility of IdoA2S residues can allow for binding to antithrombin to display anticoagulant activity⁹ and fibroblast growth factors to regulate cell growth¹⁰.

The synthesis of HS oligosaccharides remains a challenge. A number of oligosaccharides can be synthesized via a purely organic synthetic approach, but it is still very difficult to synthesize oligosaccharides larger than hexasaccharides with complex sulfation patterns. As an alternative approach, a chemoenzymatic method to synthesize HS oligosaccharides using HS biosynthetic enzymes involving glycosyltransferases, C5-epimerase, and sulfotransferases exists^{16,17}. The method can offer high-efficiency synthesis for a wide range of oligosaccharides; however, the synthesis of certain oligosaccharide sequences is not yet possible due to a lack of understanding of the substrate specificities of HS biosynthetic enzymes.

The 3-*O*-sulfation occurs infrequently in HS, but this sulfation type is believed to be intimately linked to its biological functions. The 3-*O*-sulfation can be important for anticoagulant activity¹⁹, facilitates the entry of herpes simplex virus into host cells to establish infection²⁰, regulates axon guidance and growth of neurons¹⁸ and controls the progenitor cell expansion for salivary gland development³. Precisely how the 3-*O*-sulfated glucosamine (GlcNS3S±6S) residues play roles in contributing to the biological activity of HS is currently unknown. The GIcNS3S±6S residue is shown to be surrounded by other monosaccharide residues to form a unique sulfated saccharide sequence domain, enabling HS to exert its biological effects. For example, the GIcNS3S±6S residue existing in a pentasaccharide domain enables HS to bind to antithrombin (AT). An octasaccharide carrying the GlcNS3S±6S residue interacts with herpes simplex virus glycoprotein D. Seven isoforms of 3-OST are present in the human genome that may potentially be used to prepare different 3-*O*-sulfated oligosaccharides²⁶.

Described herein are schemes using the chemoenzymatic approach to prepare a 3-O-sulfated oligosaccharide library. It is demonstrated herein that the 3-OST-3 modification, to synthesize oligosaccharides comprising the - IdoA2S-GIcNS3S- or -IdoA2S-GIcNS3S6S- disaccharide unit, must precede the 6-*O*-sulfation step, whereas 3-OST-1 modification can occur only after 6-*O*-sulfation in order to generate the -GlcA-GlcNS3S6S- disaccharide unit. There were no interactions between 3-OST-3 and the 6-*O*-sulfo groups from the tetrasaccharide substrate, consistent with the conclusion that oligosaccharide substrates for 3-OST-3 do not require 6-*O*-sulfation. In contrast, interactions between 3-OST-1 and the 6-*O*-sulfo groups from the heptasaccharide substrate were observed, suggesting that 6-*O*-sulfation is required to bind to 3-OST-1. The distinct and unique substrate requirements between 3-OST-1 and 3-OST-3, as disclosed for the first time herein, reveal that 3-*O*-sulfated HS modified by different isoforms of 3-OST are biosynthesized through different pathways.

It was widely accepted that the 3-OST-1 enzyme is responsible for synthesizing anticoagulant HS, whereas the 3-OST-3 enzyme is not^{34,35}. The AT-binding sequences isolated so far all comprise the -GIcA-GIcNS3S6S-disaccharide repeating unit, which is a product of 3-OST-1 enzyme modification^{33,39}. This long-held belief is disputed by the instant disclosure, based on the discovery that oligosaccharides developed by the disclosed methods and are products of 3-OST-3 enzyme modification bind to AT and displays anticoagulant activity. These findings indicated that 3-OST-3 is capable of synthesizing anticoagulant HS, as long as the HS comprises the structural domain similar to that of compounds disclosed herein, including for example but not limited to compound **5.**

Also disclosed herein is the discovery that some of the develop HS compounds, or oligosaccharides, have an unexpectedly fast clearance. The fast clearance of such compounds offers a potential new short-acting anticoagulant drug candidate with reduced bleeding risk. A short-acting anticoagulant drug, which can be cleared from the circulation quickly before major bleeding effects developed, would be particularly beneficial to those patients with high bleeding risk, or an elevated risk of bleeding as compared to a normal or healthy patient/subject. Although unfractionated heparin is an anticoagulant with a short half-life, the concern is that the drug causes heparin-induced thrombocytopenia (HIT), a life-threatening side effect⁴². It has been found that short oligosaccharides smaller than 12-mers⁴³ do not bind to platelet factor 4, and thus display no risk of HIT. As hexasaccharides and octasaccharide, the compounds disclosed herein, including for example compound **5,** are expected to have very low risk of HIT.

A HS compound, or synthetic heparin analogue, as disclosed herein comprise a 3-*O*-sulfated oligosaccharide comprising six to eight disaccharide units, at least one disaccharide unit is sulfated by a 3-OST-3 enzyme, and at least one IdoA2S-GIcNS3S or IdoA2S-GlcNS3S6S disaccharide unit. As illustrated in the working examples herein, such a synthetic heparin analogue may have anticoagulant activity, including a binding affinity to antithrombin ranging from about 5 nM to about 30 nM and anti-Xa activity ranging from about 10 ngml⁻¹ to about 40 ngml⁻¹ IC₅₀. The structure or makeup of such synthetic heparin analogues is exemplified by compounds 1 to 11, and particularly 1-6, including in FIGS. 6A through 6F.

As discussed further herein, in some embodiments the synthetic heparin analogues can have an unexpectedly rapid clearance rate, particularly as compared to heparin and heparin-like compounds. For example, the clearance rate of compound 5 is at least about 50%, 75% or 100% faster than that of fondaparinux in a rat or mouse model, or about 50% to about 100%, about 60% to about 90%, about 70% to about 80%, about 50% to about 75%, or about 75% to about 100%. Such rapid clearance can make such compounds suitable as short-acting anticoagulant compounds, which can be particularly suitable for applications and/or subjects at higher risk of bleeding. Such compounds are also shown not to cause heparin-induced thrombocytopenia (HIT).

Methods of synthesizing the synthetic heparin analogues are illustrated (see, e.g. FIGS. 1A, 1B and 8C) and discussed further herein, but can in some aspects include providing a saccharide substrate, elongating the saccharide substrate to a saccharide of a desired or predetermined length, performing at least one sulfation reaction using a 3-OST-3 isoform of a 3-O-sulfotransferase (3-OST) enzyme, whereby a synthetic heparin analogue is synthesized. The saccharide substrate may comprise at least one IdoA2S-GIcNS3S disaccharide unit.

The described methods of synthesizing the synthetic heparin analogues can provide surprisingly high yields of the heparin compounds. By way of example, the described methods of synthesizing the synthetic heparin analogues can have yields of greater than about 20%, greater than about 30%, greater than about 40%, greater than about 50%, about 20% to about 50%, about 30% to about 50%, or about 40% to about 50%.

Where the saccharide substrate comprises a IdoA2S-GlcNS3S±6S disaccharide unit, such a method can further comprise a 6-O-sulfation step using a 6-O-sulfotransferase (6-OST), wherein a 3-*O*-sulfation by 3-OST-3 occurs prior to the 6-*O*-sulfation step. In contrast, where the saccharide substrate comprises a GlcA-GlcNS3S6S disaccharide unit, such a method can further comprise a 6-O-sulfation step using a 6-O-sulfotransferase (6-OST), wherein a 3-*O*-sulfation by 3-OST-1 occurs prior to the 6-*O*-sulfation step.

The development of the presently disclosed heparin compounds and synthetic heparin analogues also provides for treatments and methods of treating subjects using the same. In some embodiments methods of treating subjects in need of anticoagulant therapy are provided. Such methods can comprise providing a subject in need of anticoagulant therapy and administering to the subject a synthetic heparin analogue according to the invention. Where needed the subject can also be monitored for heparin-induced thrombocytopenia, and if detected the subject can be administered an antidote to reverse the anticoagulant activity of the synthetic heparin analogue if the subject suffers from heparin-induced thrombocytopenia. Such reversal of anticoagulant activity can be achieved, for example, by andexanet alfa (AndexXa^{®}, Portola Pharmaceuticals, South San Francisco, California, United States of America) at a rate of 50% or more in the presence of 20ug/ml or less of andexanet alfa.

Thus, in accordance with some embodiments of the presently disclosed subject matter, provided is a method of treating a subject in need of anticoagulant therapy. In some embodiments, the method comprises: providing a subject in need of anticoagulant therapy; and administering to the subject a synthetic heparin analogue according to the invention.

In some embodiments, the presently disclosed subject matter provides a synthetic heparin analogue according to the invention for use in treating and/or preventing a disease or disorder wherein anticoagulant activity is advantageous. By way of example and not limitation, such diseases or disorders can include patients and individuals, including cancer patients, with high risk of deep vein thrombosis.

Example synthetic heparin analogues, or HS compounds, are disclosed herein, including for example in FIGS. 1A and 6A through 6F. In the structures shown "R" can comprise a proton (-H), -CH₃, -CH₂CH₃, or other substituted group similar to p-nitrophenyl. Such groups can also comprise, and/or be referred to as alkyl or lower alkyl. Other substituted groups similar to p-nitrophenyl can in some embodiments comprise aryl or substituted aryl. Alternatively or in addition, in some embodiments "R" can comprise a detectable tag or detectable moiety.

### Definitions

The terminology used herein is for the purpose of describing particular embodiments only and is not intended to be limiting of the presently disclosed subject matter.

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

In describing the presently disclosed subject matter, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the invention and the claims.

Following long-standing patent law convention, the terms "a", "an", and "the" refer to "one or more" when used in this application, including the claims. Thus, for example, reference to "a cell" includes a plurality of such cells, and so forth.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of a composition, dose, sequence identity (*e.g*., when comparing two or more nucleotide or amino acid sequences), mass, weight, temperature, time, volume, concentration, percentage, *etc.,* is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods or employ the disclosed compositions.

The term "comprising", which is synonymous with "including" "containing" or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of a claim to the specified materials or steps, plus those that do not materially affect the basic and novel characteristic(s) of the claimed subject matter.

With respect to the terms "comprising", "consisting of", and "consisting essentially of", where one of these three terms is used herein, the presently disclosed and claimed subject matter can include the use of either of the other two terms.

As used herein, the term "and/or" when used in the context of a listing of entities, refers to the entities being present singly or in combination. Thus, for example, the phrase "A, B, C, and/or D" includes A, B, C, and D individually, but also includes any and all combinations and subcombinations of A, B, C, and D.

As used herein the term "alkyl" refers to C₁₋₂₀ inclusive, linear (*i.e.,* "straight-chain"), branched, or cyclic, saturated or at least partially and in some cases fully unsaturated (*i.e.,* alkenyl and alkynyl) hydrocarbon chains, including for example, methyl, ethyl, propyl, isopropyl, butyl, isobutyl, *tert-*butyl, pentyl, hexyl, octyl, ethenyl, propenyl, butenyl, pentenyl, hexenyl, octenyl, butadienyl, propynyl, butynyl, pentynyl, hexynyl, heptynyl, and allenyl groups. "Branched" refers to an alkyl group in which a lower alkyl group, such as methyl, ethyl or propyl, is attached to a linear alkyl chain. "Lower alkyl" refers to an alkyl group having 1 to about 8 carbon atoms (*i.e.,* a C₁₋₈ alkyl), e.g., 1, 2, 3, 4, 5, 6, 7, or 8 carbon atoms. "Higher alkyl" refers to an alkyl group having about 10 to about 20 carbon atoms, e.g., 10, 11, 12, 13, 14, 15, 16, 17, 18, 19, or 20 carbon atoms. In certain embodiments, "alkyl" refers, in particular, to C₁₋₈ straight-chain alkyls. In other embodiments, "alkyl" refers, in particular, to C₁₋₈ branched-chain alkyls.

Alkyl groups can optionally be substituted (a "substituted alkyl") with one or more alkyl group substituents, which can be the same or different. The term "alkyl group substituent" includes but is not limited to alkyl, substituted alkyl, halo, arylamino, acyl, hydroxyl, aryloxyl, alkoxyl, alkylthio, arylthio, aralkyloxyl, aralkylthio, carboxyl, alkoxycarbonyl, oxo, and cycloalkyl. There can be optionally inserted along the alkyl chain one or more oxygen, sulfur or substituted or unsubstituted nitrogen atoms, wherein the nitrogen substituent is hydrogen, lower alkyl (also referred to herein as "alkylaminoalkyl"), or aryl.

Thus, as used herein, the term "substituted alkyl" includes alkyl groups, as defined herein, in which one or more atoms or functional groups of the alkyl group are replaced with another atom or functional group, including for example, alkyl, substituted alkyl, halogen, aryl, substituted aryl, alkoxyl, hydroxyl, nitro, amino, alkylamino, dialkylamino, sulfate, and mercapto.

The term "aryl" is used herein to refer to an aromatic substituent that can be a single aromatic ring, or multiple aromatic rings that are fused together, linked covalently, or linked to a common group, such as, but not limited to, a methylene or ethylene moiety. The common linking group also can be a carbonyl, as in benzophenone, or oxygen, as in diphenylether, or nitrogen, as in diphenylamine. The term "aryl" specifically encompasses heterocyclic aromatic compounds. The aromatic ring(s) can comprise phenyl, naphthyl, biphenyl, diphenylether, diphenylamine and benzophenone, among others. In particular embodiments, the term "aryl" means a cyclic aromatic comprising about 5 to about 10 carbon atoms, e.g., 5, 6, 7, 8, 9, or 10 carbon atoms, and including 5- and 6-membered hydrocarbon and heterocyclic aromatic rings.

The aryl group can be optionally substituted (a "substituted aryl") with one or more aryl group substituents, which can be the same or different, wherein "aryl group substituent" includes alkyl, substituted alkyl, aryl, substituted aryl, aralkyl, hydroxyl, alkoxyl, aryloxyl, aralkyloxyl, carboxyl, acyl, halo, nitro, alkoxycarbonyl, aryloxycarbonyl, aralkoxycarbonyl, acyloxyl, acylamino, aroylamino, carbamoyl, alkylcarbamoyl, dialkylcarbamoyl, arylthio, alkylthio, alkylene, and -NR'R", wherein R' and R" can each be independently hydrogen, alkyl, substituted alkyl, aryl, substituted aryl, and aralkyl.

Thus, as used herein, the term "substituted aryl" includes aryl groups, as defined herein, in which one or more atoms or functional groups of the aryl group are replaced with another atom or functional group, including for example, alkyl, substituted alkyl, halogen, aryl, substituted aryl, alkoxyl, hydroxyl, nitro, amino, alkylamino, dialkylamino, sulfate, and mercapto.

Specific examples of aryl groups include, but are not limited to, cyclopentadienyl, phenyl, furan, thiophene, pyrrole, pyran, pyridine, imidazole, benzimidazole, isothiazole, isoxazole, pyrazole, pyrazine, triazine, pyrimidine, quinoline, isoquinoline, indole, carbazole, and the like.

A structure represented generally by a formula such as: 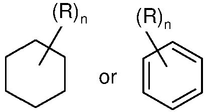 as used herein refers to a ring structure, for example, but not limited to a 3-carbon, a 4-carbon, a 5-carbon, a 6-carbon, and the like, aliphatic and/or aromatic cyclic compound comprising a substituent R group, wherein the R group can be present or absent, and when present, one or more R groups can each be substituted on one or more available carbon atoms of the ring structure. The presence or absence of the R group and number of R groups is determined by the value of the integer n. Each R group, if more than one, is substituted on an available carbon of the ring structure rather than on another R group. For example, the structure: 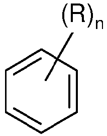 wherein n is an integer from 0 to 2 comprises compound groups including, but not limited to: 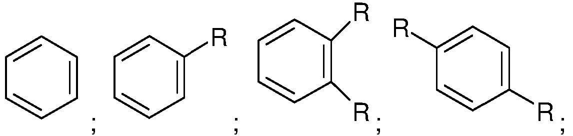 \and the like.

"Cyclic" and "cycloalkyl" refer to a non-aromatic mono- or multicyclic ring system of about 3 to about 10 carbon atoms, e.g., 3, 4, 5, 6, 7, 8, 9, or 10 carbon atoms. The cycloalkyl group can be optionally partially unsaturated. The cycloalkyl group also can be optionally substituted with an alkyl group substituent as defined herein, oxo, and/or alkylene. There can be optionally inserted along the cyclic alkyl chain one or more oxygen, sulfur or substituted or unsubstituted nitrogen atoms, wherein the nitrogen substituent is hydrogen, alkyl, substituted alkyl, aryl, or substituted aryl, thus providing a heterocyclic group. Representative monocyclic cycloalkyl rings include cyclopentyl, cyclohexyl, and cycloheptyl. Multicyclic cycloalkyl rings include adamantyl, octahydronaphthyl, decalin, camphor, camphane, and noradamantyl.

The term "heterocycle" refers to a non-aromatic or aromatic, monocyclic or multicyclic ring system of about 3 to about 14 atoms, wherein at least one of the atoms is a heteroatom (e.g., oxygen, nitrogen, or sulfur). The term "N-heterocycle" refers to a heterocycle wherein at least one of the heteroatoms is a nitrogen atom. Examples of N-heterocycles include, but are not limited to, azetidine, pyrrolidine, pyrrole, pyrroline, piperidine, pyridine, piperazine, pyrazine, pyrimidine, pyridazine, morpholine, and thiazine.

"Aralkyl" refers to an aryl-alkyl- group wherein aryl and alkyl are as previously described, and included substituted aryl and substituted alkyl. Exemplary aralkyl groups include benzyl, phenylethyl, and naphthylmethyl.

As used herein, the term "acyl" refers to an organic carboxylic acid group wherein the -OH of the carboxyl group has been replaced with another substituent (i.e., as represented by RC(=O)-, wherein R is an alkyl, substituted alkyl, aralkyl, aryl or substituted aryl group as defined herein). As such, the term "acyl" specifically includes arylacyl groups, such as an acetylfuran and a phenacyl group. Specific examples of acyl groups include acetyl and benzoyl.

"N-acyl" refers to a group having the structure -N-C(=O)-R, wherein R is as defined for acyl. These groups can also be referred to as amides. Modified N-acyl groups include compounds wherein the oxygen of the N-acyl has been replaced by S or NH, as well as to compounds wherein the carbonyl group (i.e., the -C(=O)-) is attached to a second heteroatom in addition to the nitrogen. For example, the carbonyl can be attached to a second nitrogen atom to form a urea linkage (i.e., -NH-C(=O)-NH-R).

The term "amino" refers to the -NH₂, the -NHR, and the -NR₂ groups, wherein each R is independently alkyl, substituted alkyl, aryl, substituted aryl, or aralkyl, as well as to amino and ammonium functionalities in N-heterocycles (e.g., morpholine, etc). As used herein the term "amino" can also refer to substituents that provide quaternary ammonium cations, such as -⁺NH₃, - ⁺NH(R)₂, and -⁺N(R)₃ groups, wherein each R is independently alkyl, substituted alkyl, aryl, substituted aryl or aralkyl.

The term "ester" refers to a moiety comprising an -O-C(=O)-R group, wherein R can be alkyl, substituted alkyl, aralkyl, aryl, or substituted aryl. In some embodiments, the R group can include an amino substituent and the ester is an amino ester.

The term "amide" refers to a moiety comprising a -N(R')-C(=O)-R group, wherein R is selected from alkyl, substituted alkyl, aralkyl, aryl or substituted aryl and R' is H, alkyl, substituted alkyl, aralkyl, aryl, or substituted aryl.

The term "urea" as used herein can refer to a moiety comprising a - N(R')-C(=O)-N(R')- group, wherein each R' is independently H, alkyl, substituted alkyl, aralkyl, aryl, or substituted aryl.

The term "hydroxyl" refers to the -OH group.

When the term "independently selected" is used, the substituents being referred to (e.g., R groups, such as groups R₁ and R₂, or groups X and Y), can be identical or different. For example, both R₁ and R₂ can be substituted alkyls, or R₁ can be hydrogen and R₂ can be a substituted alkyl, and the like.

### Subjects

The subject treated, screened, tested, or from which a sample is taken, is desirably a human subject, although it is to be understood that the principles of the disclosed subject matter indicate that the compositions and methods are effective with respect to invertebrate and to all vertebrate species, including mammals, which are intended to be included in the term "subject". Moreover, a mammal is understood to include any mammalian species in which screening is desirable, particularly agricultural and domestic mammalian species.

The disclosed methods, compounds and treatments are particularly useful in the testing, screening and/or treatment of warm-blooded vertebrates. Thus, the presently disclosed subject matter concerns mammals and birds.

More particularly, provided herein is the testing, screening and/or treatment of mammals such as humans, as well as those mammals of importance due to being endangered (such as Siberian tigers), of economical importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), and horses. Also provided is the treatment of birds, including the treatment of those kinds of birds that are endangered, kept in zoos, as well as fowl, and more particularly domesticated fowl, i.e., poultry, such as turkeys, chickens, ducks, geese, guinea fowl, and the like, as they are also of economical importance to humans. Thus, provided herein is the treatment of livestock, including, but not limited to, domesticated swine (pigs and hogs), ruminants, horses, poultry, and the like.

In some embodiments, the subject to be used in accordance with the presently disclosed subject matter is a subject in need of treatment and/or diagnosis. In some embodiments, a subject can be in need of anticoagulant therapy or related condition or phenotype. In some embodiments, the subject in need of anticoagulant therapy can be a subject with a high risk of bleeding.

### Formulations

The compositions of the presently disclosed subject matter comprise in some embodiments a composition that includes a pharmaceutically acceptable carrier. Any suitable pharmaceutical formulation can be used to prepare the adenovirus vectors for administration to a subject.

For example, suitable formulations can include aqueous and nonaqueous sterile injection solutions which can contain anti-oxidants, buffers, bacteriostats, bactericidal antibiotics and solutes which render the formulation isotonic with the bodily fluids of the intended recipient; and aqueous and nonaqueous sterile suspensions which can include suspending agents and thickening agents. The formulations can be presented in unit-dose or multidose containers, for example sealed ampoules and vials, and can be stored in a frozen or freeze-dried (lyophilized) condition requiring only the addition of sterile liquid carrier, for example water for injections, immediately prior to use. Some exemplary ingredients are SDS, mannitol or another sugar, and phosphate-buffered saline (PBS).

It should be understood that in addition to the ingredients particularly mentioned above the formulations of this presently disclosed subject matter can include other agents conventional in the art having regard to the type of formulation in question. For example, sterile pyrogen-free aqueous and nonaqueous solutions can be used.

### Administration

Administration of the compositions of the presently disclosed subject matter can be by any method known to one of ordinary skill in the art, including, but not limited to intravenous administration, intrasynovial administration, transdermal administration, intramuscular administration, subcutaneous administration, topical administration, rectal administration, intravaginal administration, intratumoral administration, oral administration, buccal administration, nasal administration, parenteral administration, inhalation, and insufflation. In some embodiments, suitable methods for administration of a composition of the presently disclosed subject matter include, but are not limited to intravenous. Alternatively, a composition can be deposited at a site in need of treatment in any other manner. The particular mode of administering a composition of the presently disclosed subject matter depends on various factors.

### Dosage

An effective dose of a composition of the presently disclosed subject matter is administered to a subject in need thereof. A "therapeutically effective amount" is an amount of the composition sufficient to produce a measurable response (*e.g*., anticoagulation). In some embodiments, a therapeutically effective amount is an amount sufficient to prevent coagulation, i.e. anticoagulation. In some embodiments, a therapeutically effective amount is an amount sufficient to improve the health, well-being, prognosis and/or survivability of a subject requiring anticoagulant therapy. Actual dosage levels of active ingredients in the compositions of the presently disclosed subject matter can be varied so as to administer an amount of the active compound(s) that is effective to achieve the desired therapeutic response for a particular subject. The selected dosage level can depend upon the activity of the therapeutic composition, the route of administration, combination with other drugs or treatments, the severity of the condition being treated, and the condition and prior medical history of the subject being treated. However, it is within the skill of the art to start doses of the compositions at levels lower than required to achieve the desired therapeutic effect and to gradually increase the dosage until the desired effect is achieved.

After review of the disclosure of the presently disclosed subject matter presented herein, one of ordinary skill in the art can tailor the dosages to an individual patient, taking into account the particular formulation, method for administration to be used with the composition, and severity of the condition. Further calculations of dose can consider patient height and weight, severity and stage of symptoms, and the presence of additional deleterious physical conditions. Such adjustments or variations, as well as evaluation of when and how to make such adjustments or variations, are well known to those of ordinary skill in the art of medicine.

### EXAMPLES

The following Examples are included to further illustrate various embodiments of the presently disclosed subject matter.

### MATERIALS AND METHODS

### Expression and purification of enzymes

The expression of 6-*O*-sulfotransferase 1 (6-OST-1) and 6-*O-*sulfotransferase 3 (6-OST-3) was carried out in the Sf9 cells (Invitrogen) using Sf-900^{™} III SFM (Life Technologies). Insect cells at a concentration of 2.0 × 10⁶ cells/mL were infected by the recombinant virus expressing mouse 6-OST-1 and human 6-OST-3 and were allowed to incubate in the shaker at 27°C for 96 h. The culture solution was centrifuged at 4,000 RPM for 10 min to pellet the cells. The supernatant containing 1mM phenylmethanesulfonyl fluoride (PMSF, a 100 mM stock solution was freshly made in 95% ethanol), 0.05% Triton X-100 and 0.2% glycerol was then centrifuged at 8000 RPM for 30 min and filtered through a 1.5 µm membrane. The resultant medium was then mixed with an equal volume of 40 mM 3-(N-morpholino) propanesulfonic acid (MOPS) buffer containing 0.05% Triton-100 and 2% Glycerol, pH 7.0). Heparin Toyopearl gel (Tosoh Bioscience) column was used to purify the 6-OST-1 and 3 with two buffers: buffer A contained 20 mM MOPS, pH 7.0, 100 mM NaCl, 2% glycerol and 0.1% reduced triton X-100 (Sigma); and buffer B contained 20 mM MOPS, pH 7.0, 1 M NaCl, 2% glycerol and 0.1% reduced triton X-100. After loading the medium, buffer A was used to wash the column until UV absorbance at 280 nm reached baseline at a flow rate of 4 mL/min. A gradient elution of 0-100% B in 60 min was applied, and the column was eluted with additional 60 min with100% B at a flow rate of 1.5 mL/min. The purification of enzyme was performed at 4°C.

The expression of 3-OST-1 and 3-OST-3 was performed in *Escherichia coli* using BL21 cells. The transformed cells were grown in LB medium containing 50 µg/L kanamycin for 3-OST-1 and 3-OST-3 and incubated at 37°C until the OD600 reached 0.6-0.8. Isopropylthiogalactopyranoside (IPTG) at a final concentration of 0.2 mM was added to induce the expression of 3-OST-1 and 3. The bacteria culture was kept shaking at 22°C overnight. The bacterial cells were harvested by spinning at 3400 RPM for 15 min. The cells were resuspended in 25 mL of buffer solution containing 25 mM Tris, pH 7.5, 30 mM imidazole, and 500 mM NaCl. The suspension was sonicated and centrifuged at 14,000 RPM for 30 min. The supernatant was filtered through a 0.45 µm membrane before purification. Nickel-agarose (GE Healthcare) column was used to purify the proteins with two buffers: buffer C contained 25 mM Tris, pH 7.5, 30 mM imidazole, and 500 mM NaCl; buffer D contained 25 mM Tris, pH 7.5, 300 mM imidazole, and 500 mM NaCl. After loading the medium, buffer C was used to wash the column until UV absorbance at 280 nm reached baseline at a flow fate of 2 mL/min. Buffer D was then applied to elute the protein.

### Chemoenzymatic synthesis of oligosaccharides (compounds 1 to 6)

The synthesis of compound **1** to **3** was initiated from a hexasaccharide (GlcNS-GlcA-GlcNS-IdoA2S-GlcNS-GlcA-pNP), designated as "NS2S 6-mer substrate". To synthesize compound **1,** the substrate (25 mg) was incubated with 1.8 mM 3'-phosphoadenosine 5'-phosphosulfate (PAPS) in a buffer containing 50 mM MOPS (pH 7.0), 10 mM MnCl2, 7 mM MgCl2, and 2 mL 3-OST-3 (0.11 mg/mL) in a total volume of 100 mL. The reaction mixture was incubated at 37°C overnight. The completion of the reaction was monitored by injecting a small amount of reaction mixture to anion exchange HPLC (TSKgel DNA-NPR-column (4.6 mm×7.5 cm, 2.5 µm, from Tosoh Bioscience)). If reaction was <60% complete, additional 3-OST-3 enzyme and PAPS were added, and the reaction mixture was kept at 37°C for another 18-24 hours. If the reaction was completed, the reaction mixture was subjected to Q-Sepharose chromatography (GE Healthcare).

To synthesize compound **2,** compound **1** (5 mg) was incubated with 6-OST-1, 6-OST-3 enzymes and 1.3 mM PAPS in a buffer containing 100 mM MOPS (pH 7.0), and 1 mL enzyme cocktails of 6-OST-1 and 3 in a total volume of 100 mL at 37°C overnight. The completion of the reaction was monitored by injecting a small amount of reaction mixture to anion exchange HPLC, and the product was purified by Q-Sepharose. To synthesize compound **3,** compound **2** (4 mg) was incubated with 1.3 mM PAPS in a buffer containing 18 mM MOPS (pH 7.0), 5 mM MnCl2, 5 mM MgCl2, and 6 mL 3-OST-1 (4 µg/mL) in a total volume of 100 mL. The reaction mixture was incubated at 37°C overnight. The completion of the reaction was monitored by injecting a small amount of reaction mixture to anion exchange HPLC, and the product was purified by Q-Sepharose.

The synthesis of compound **4** to **6,** an octasaccharide (GIcNS-GIcA-GlcNS-IdoA2S-GlcNS-IdoA2S-GlcNS-GlcA-pNP), designated as NS2S 8-mer substrate, was used as the starting material. To synthesize compound **4,** 30 mg of the substrate was incubated with 2 mM PAPS in a buffer containing 33 mM MOPS (pH 7.0), 10 mM MnCl2, 5 mM MgCl2, and 4 mL 3-OST-3 (0.11 mg/mL) in a total volume of 90 mL. The reaction mixture was incubated at 37°C overnight. The completion of the reaction was monitored by injecting a small amount of the reaction mixture to anion exchange HPLC. If reaction was <60% complete, additional 3-OST-3 enzyme and PAPS were added, and the reaction mixture was kept at 37°C for another 18-24 hours. If the reaction was completed, the reaction mixture was subjected to Q-Sepharose chromatography (GE Healthcare).

To synthesis of compound **5,** compound **4** (22 mg) was incubated with 6-OST-1, 6-OST-3 enzymes and 0.8 mM PAPS in a buffer containing 100 mM MOPS (pH 7.0), and 3 mL a mixture of 6-OST-1 and 3 in a total volume of 100 mL at 37°C overnight. The completion of the reaction was monitored by injecting a small amount of reaction mixture to anion exchange HPLC, and the product was purified by Q-Sepharose. To synthesize compound **6,** compound **5** (6.5 mg) was incubated with 1.3 mM PAPS in a buffer containing 18 mM MOPS (pH 7.0), 5 mM MnCl2, 5 mM MgCl2, and 4.5 mL 3-OST-1 in a total volume of 100 mL (4 µg/mL). The reaction mixture was incubated at 37°C overnight. The completion of the reaction was monitored by injecting a small amount of reaction mixture to anion exchange HPLC, and the product was purified by Q-Sepharose.

### Substrate specificities of 3-OST-1 and 3-OS T-3

To determine the substrate requirements for 3-OST-1 and 3-OST-3, different sizes of structurally homogeneous oligosaccharides were used. These oligosaccharides included N-sulfated and 2-O-sulfated 6-mers to 12-mers (6-mer2S to 12-mer2S) and N-sulfated, 2-O-sulfated and 6-O-sulfated 6-mers to 12-mers (6-mer2S6S to 12-mer2S6S). These oligosaccharides were prepared by the chemoenzymatic method 1, and their structures are displayed in FIG. 5C. Oligosaccharides (0.033 mM) were incubated in the 100 µL reaction buffer containing 50 mM MOPs (pH 7.0), 10 mM MnCl2, 5mM MgCl2, 2.5 µL 3-OST-1 or 3-OST-3, and 30 µM of PAPS mixed with [35S] PAPS (1-3×10⁵ cpm) at 37°C for 1 h. To the reaction mixture, 900 µL 3 M urea containing 1.4 mM EDTA, 50 mM sodium acetate and 150 mM NaCl was added to quench the reaction. The reaction mixture was then purified using the DEAE-column.

### Kinetic analysis of 3-OST-3 towards different oligosaccharide substrates

The enzyme kinetic was characterized by incubating the mixture of 30 µL of purified 3-OST-3 for oligosaccharides with *N*-sulfation and 2-*O*-sulfation or 60 µL of purified 3-OST-3 for oligosaccharides with *N*-sulfation, 2-*O-*sulfation and 6-*O*-sulfation. PAPS (120 µM) was mixed with 1×10⁵ cpm of [35S] PAPS as sulfate donor and oligosaccharides at various concentrations from 0-200 µM at 37°C for 1h. The reaction was loaded on a DEAE column to purify the 35S-labeled oligosaccharide product. The amount of 35S-labeled oligosaccharide products were plotted against concentrations of substrate followed by curve-fitting for Michalis-Menten graph using Sigma Plot software to obtain *Km* and *Vmax* values.

### Purification of compound 1 to 6 by Q-Sepharose

The purification of sulfated oligosaccharide was performed with Q-Sepharose column. Mobile phase A was 25 mM Tris, pH 7.5. Mobile phase B contained 25 mM Tris and 1 M NaCl, pH 7.5. The elution gradient was based on the sulfate group numbers of synthesized oligosaccharides with a flow rate of 1 mL/min. The absorption at 310 nm and 260 nm was scanned and recorded. After purification, the sample was dialyzed twice using 1000 MWCO membrane against a buffer containing 5 mM sodium phosphate dibasic (pH 7.5).

### HPLC analysis of synthesized oligosaccharides

TSKgel DNA-NPR-column was applied to detect the degree of completion of the reaction and the purity of synthesized oligosaccharide after purification. Mobile phase A was 25 mM Tris, pH 7.5. Mobile phase B was 25 mM Tris and 1 M NaCl, pH 7.5. The gradient step was 0-100% B in 100 min with a flow rate of 0.4 mL/min. The absorption at 310 nm and 260 nm was used to monitor the eluent.

### ESI-MS analysis of oligosaccharides

Molecular weight conformation of synthesized oligosaccharides was determined by the ESI-MS (Thermo LCQ-Deca). ESI-MS analysis was performed in the negative ion mode and with the following parameters: I Spray voltage at 3.0 kV, curved desolvation line temperature at 120°C. The mass range was set at 300-1000.

### Preparation of 3-O-[34S]sulfated compound 4

To synthesize 3-*O*-[34S] sulfated compound **4,** the substrate NS2S 8-mer (2 mg) was incubated with 3-OST-3 enzyme (0.11 mg/mL) and 0.1 mM [34S]PAPS in a buffer containing 38 mM MOPS (pH 7.0), 10 mM MnCl2, 5 mM MgCl2, and 6 mL 3-OST-3 in a total volume of 20 mL. The reaction mixture was incubated at 37°C overnight. The purification of product was performed with Q-Sepharose column.

### Tandem MS analysis of 3-OST-3 modified octasaccharide

The tandem mass spectra analysis was acquired on a Thermo LTQ-FT instrument in negative ion mode with the following instrument parameters: I spray voltage (kV), -3.5 kV; capillary voltage (kV), -40 kV; tube lens (V), -50 V and capillary temperature at 275°C. For the tandem mass, the selected precursor ions were performed with the following parameters: Iso Width (m/z): 3.0, Normalized Collision Energy (%): 50.0, Act. Q: 0.250, Act. Time: 30, Max. Inject Time (ms): 500.000. The MS and MS/MS data were recorded and processed by using Xcalibur 2.2 software.

### Structural analysis of compound 1 to 6 by NMR

NMR experiments were performed at 298 K on Bruker Avance 700 MHz and 850 MHz spectrometer with Topsin 3.2 software. Samples (0.5 to 3.0 mg) were each dissolved in 0.5 ml D₂O (99.996%, Sigma-Aldrich) and lyophilized three times to remove the exchangeable protons. The samples were re-dissolved in 0.5 ml D₂O and transferred to NMR microtubes (O.D. 5 mm, Norrell). Chemical shifts are referenced to external 2,2-dimethyl-2-silapentane-5-sulfonate sodium salt (DSS, Sigma, Co.). Deuterated EDTA (Sigma, Co.) was added to remove the effect of paramagnetic ions. 1D 1H-NMR experiments "zg" pulse sequence were performed with 64 scans and an acquisition time of 3.8 sec. 1D 13C-NMR experiments "zgdc30" pulse sequence were performed with 10,000 scans and an acquisition time of 1.0 sec. 2D 1H-13C HSQC experiments "hsqcgpph" pulse sequence were performed with 48 scans, 512 increments, 1.5 sec relaxation delay, and 120 msec acquisition time. 2D spectra were recorded with GARP carbon decoupling. 48 dummy scans were used prior to the start of acquisition. 2048 total points were collected in f2. ¹³C transmitter offset was set at 90.0 ppm.

### Molecular dynamics pre-processing

An existing crystal structure of the AT-pentasaccharide complex (PDB ID: 3EVJ) was employed as a starting structure of the system⁴⁷. Amino acid residues 25-33 and 396 were not resolved in the crystal structure, so were generated with the Chimera interface to Modeller⁴⁸⁻⁵¹. The *N*-glycans were removed, as they are distal (> 15 Å) from the fondaparinux ligand. The original ligand was modified for the various simulations with tleap and manually adjusted with Chimera. Parameters for the amino acids and carbohydrate residues were obtained from the ff14SB7 and GLYCAM06 (J-1) force fields^{53,54}. The system was neutralized with Na+ ions, and solvated with the TIP3P water model in a truncated octahedral box 12 Å around the complex.

### Molecular dynamics protocol

Energy minimizations and MD simulations were performed using the *pmemd.cuda* module from AMBER14⁵². The Cα atoms of the protein backbone were restricted with cartesian restraints (10 kcal/mol Å²) throughout every step of the process. Two of the simulations required internal restraints on IdoA2S residue d (FIG. 4A) to maintain a *¹C₄* or *²S_{O}* conformation, and were implemented according to settings described previously⁵⁵. The systems were minimized using the steepest descent method for the initial 1000 cycles before switching to conjugate gradient for the remaining 24,000 cycles. To aid in generating a stable configuration for the AT-oligosaccharide complexes, two minimizations were performed with varying atomic restraints. Initially, every solute atom was restrained. Subsequently, the restraints on the ligand and side chains of the protein were removed. Electrostatic interactions were treated with the Particle-Mesh Ewald algorithm⁵⁶ and an 8 Å cutoff for non-bonded interactions was employed. The SHAKE algorithm was applied to hydrogen-containing bonds, enabling an integration time step of 2 fs. The system was heated to 300 K under NVT conditions over 60 ps by employing the Berendsen thermostat with a coupling time constant of 1 ps, and allowed to equilibrate for a total of 50 ns under NPT conditions. Post-equilibration data sets were collected for an additional 150 ns, also under NPT conditions.

### Simulation data analysis

Interaction energies were calculated with the single-trajectory Molecular Mechanics-Generalized Born Solvent Accessible Surface Area (MM-GBSA) method⁵⁷ using the MPBSA.py.MPI module⁵². Before the analyses, all water molecules and ions were removed from each complex, and the contribution from desolvation energy approximated through the GB implicit solvation model (igb=2)⁵⁸. The simulation was divided into 5 ns bins and average interaction energy contributions computed from an ensemble of 100 snapshots evenly distributed within each bin. Statistical analysis was performed with GraphPad Prism for Windows version 5.04. Significance was determined based upon a p-value ≤ 0.0001 according to t-test analysis. Images were created with the Visual Molecular Dynamics program⁵⁹.

### Determination of anti-FXa activity in vitro

Factor Xa (Enzyme Research Laboratories, South Bend, IN) was diluted to 60 nM with PBS containing 1 mg/mL bovine serum albumin (BSA). Human antithrombin (AT) (from Cutter Biological) was prepared in PBS containing 1 mg/mL BSA at a concentration of 0.65 µM. The chromogenic substrate S-2765 (Diapharma) was dissolved in water to make a 1mg/mL stock solution. The oligosaccharides were made up of different concentration (10-200 nM) with PBS. The solution was a mixture of 60 µL of AT and 15 µL sample solution, which was vortexed and incubated at room temperature for 2 min. Factor Xa (90 µL) was then added and incubated at room temperature for 4 min, following which 30 µL S-2765 was added. The reaction mixture was measured based on their absorbance at 405 nm continuously for 2 min. The calculation of IC₅₀ values was plotted as a function of sample concentrations versus the initial reaction rates.

### Determination of anti-FXa effect in vivo

All studies were performed under an Institutional Animal Care and Use Committee (IACUC)-approved protocol at the University of North Carolina, following Public Health Service guidelines for laboratory animal care and use. Sixteen 400-g Lewis rats were equally divided into four groups. Each group was intravenously administered with either endotoxin-free fondaparinux (0.46 µM kg-1), compound **11** (0.43 µM kg⁻¹), compound **5** (0.42 µM kg⁻¹) or saline. Blood was then drawn at specified time points (0.5, 1, 2, 4, 8 hours) after the compounds had been administered, with one sample taken just before administration. 0.8 mL of blood was drawn from the contralateral femoral/saphenous vein into 150 mM citrate at 0.5, 1, 2, 4 and 8 hours following injection of the compounds, with one sample drawn immediately prior to injection. The samples were centrifuged to obtain approximately 400 µL of plasma. The blood samples were subjected to FXa activity analysis. Saline was given periodically subcutaneously to maintain fluid volume.

### Determination of the clearance of oligosaccharides from the drug-treated animals

Using a calibration curve generated for each compound, the concentration of compound in the reaction mixture was read off for each corresponding FXa activity percentage. The average plasma concentration and standard deviation between the 4 rats in each group were calculated for each time point; these values were used to plot a graph of plasma concentration against time.

### EXAMPLE 1

### Chemoenzymatic synthesis of oligosaccharides carrying GlcNS3S and GlcNS3S6S residues

The synthesis of hexasaccharides (compound **1** to **3,** FIG. 1A) and octasaccharides (compound **4** to **6,** FIG. 1A) was accomplished in the current study. Two 3-O-sulfotransferase (3-OST) isoforms, 3-OST-1 and 3-OST-3, were employed to install the GIcNS3S±6S residue into different saccharide sequences. The 3-OST-1 enzyme introduces a sulfation to form a GIcNS3S6S residue that is linked to a GIcA residue at the nonreducing end, forming the disaccharide unit of -GlcA-GlcNS3S6S-; whereas the 3-OST-3 enzyme introduces a sulfation to form a GlcNS3S residue that is linked to an IdoA2S residue at the nonreducing end, forming the disaccharide unit of -IdoA2S-GIcNS3S-. Although 3-OST-1 has been successfully used to synthesize oligosaccharides in numerous studies^{16,17,27,28}, the use of 3-OST-3 to synthesize oligosaccharides comprising the -IdoA2S-GIcNS3S- disaccharide units has not been reported.

Disclosed herein is the discovery that 3-OST-3 and 3-OST-1 have different substrate requirements. The 3-OST-1 enzyme sulfates oligosaccharide substrates that carry 6-*O*-sulfation, while displaying very low reactivity towards the oligosaccharide substrates that lack 6-*O*-sulfation (FIG. 5A and 5B), which is consistent with the conclusions described previously¹⁶. In contrast, 3-OST-3 preferentially sulfates oligosaccharides that do not carry 6-*O*-sulfation (FIG. 5A and 5B), but the reactivity of those 6-*O*-sulfated oligosaccharide substrates to 3-OST-3 modification was low (FIGS. 5A, 5B and 5C). Results from kinetic analysis demonstrate that 3-OST-3 has higher catalytic efficiency for oligosaccharide substrates without 6-*O*-sulfation as determined by the values of *kcat*/*Km* (Table 1).

**Table 1. Kinetic parameters of 3-OST-3 towards oligosaccharide substrates**

| | **6mer2s** | **6mer2S6S** | **8mer2S** | **8mer2S6S** |
|---|---|---|---|---|
| *Kₘ (*µ*M)* | 364.5 | 20.7 | 86.9 | 23.1 |
| *k_{cat} (min⁻¹)* | 0.49 | 0.018 | 0.73 | 0.095 |
| *k_{cat}*/*Kₘ* | 0.013 | 0.0086 | 0.084 | 0.041 |
| 10⁵ × (M × min)⁻¹ | | | | |

| | **10mer2s** | **10mer2S6S** | **12mer2S** | **12mer2S6S** |
|---|---|---|---|---|
| *Kₘ (*µ*M)* | 44.1 | 16.6 | 31.4 | 7.7 |
| *k_{cat} (min⁻¹)* | 0.49 | 0.05 | 0.61 | 0.03 |
| *k_{cat}*/*Kₘ* | 0.1 | 0.03 | 0.2 | 0.04 |
| 10⁵ × (M × min)⁻¹ | | | | |

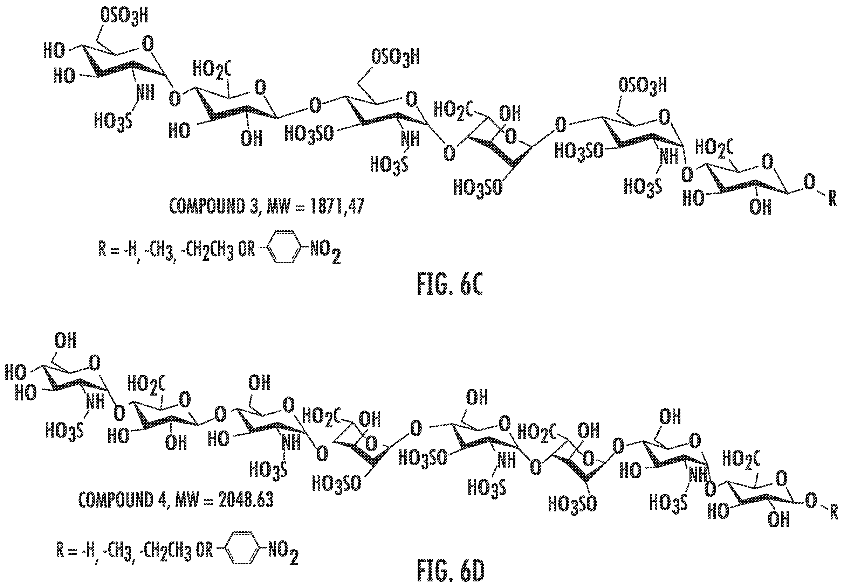

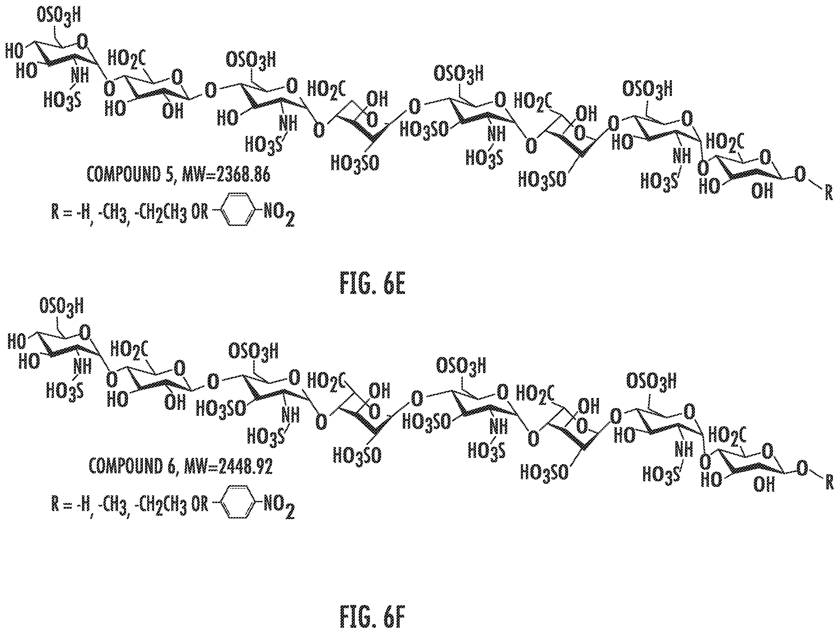

The discovery of distinct substrate requirements for 3-OST-1 and 3-OST-3 led to the development, as disclosed herein, of two separate schemes to synthesize oligosaccharides that contain different 3-*O*-sulfated saccharide sequences. For the synthesis of oligosaccharides containing the IdoA2S-GIcNS3S±6S disaccharide unit (compound **1, 2, 4** and **5,** depicted in FIGS. 6A, 6B, 6D and 6E, respectively), 3-*O*-sulfation by 3-OST-3 was introduced prior to the 6-*O*-sulfation step (FIG. 1B), whereas for the synthesis of oligosaccharides containing the GlcA-GlcNS3S6S disaccharide unit, the 3-*O-*sulfation by 3-OST-1 was performed after the 6-*O*-sulfation step (FIG. 1B) (compound **3** and **6,** depicted in FIGS. 6C and 6F). The AT-binding domain comprises a pentasaccharide unit of -GlcNS(or Ac)6S-GlcA-GlcNS3S±6S-IdoA2S-GlcA-GlcNS6S-, of which the 3-*O*-sulfation is can be needed for high binding affinity^{24,29}. Among all the oligosaccharides tested in this study, only compound **8** and **11** contain the pentasaccharide unit.

### EXAMPLE 2

### Structural and conformational analysis of oligosaccharides

The purity and structural analysis of compound **1** to **6** was conducted, representative data for the analysis of compound **6,** depicted in FIG. 6E, are shown in FIGS. 2A and 2B. Compound **6** was eluted as a single peak from high resolution anion exchange HPLC, suggesting that the compound is pure (FIG. 2A). The molecular mass of compound **6** was determined to be 2449.43 ± 0.74 by electrospray ionization mass spectrometry (ESI-MS), which is very close to the calculated molecular mass of 2448.92 (FIG. 2B). The 1H-NMR spectrum of compound **6** clearly shows eight anomeric protons, confirming that the product is an octasaccharide. The 13C-NMR and full NMR assignment for compound **6** are shown in Supplementary FIG. 25 and Supplementary Table 1, respectively. To locate the 3-*O*-sulfo group in compound **4,** tandem MS analysis was carried out. In this analysis, a stable isotopically labeled [34S]sulfo group was introduced by the 3-OST-3 enzyme, allowing us to unambiguously identify the presence of the 3-*O*-sulfo group at residue d in compound **4.** As compound **4** was the intermediate for compound **5** and **6,** the tandem MS analysis of compound **4** also helped to locate the IdoA2S-GIcNS3S6S disaccharide unit in compound **5** and **6.**

The pyranose rings of IdoA2S residues interconvert between different conformations, including chair forms (*1C4* and *4C1*) and the skew boat form (*2SO*) (FIG. 1C). The impact of 3-*O*-sulfation on the conformation of neighboring IdoA2S residues was investigated. The conformational analysis was completed by NMR through measuring the three bond proton-proton coupling constants (*3JH-H*) ³⁰. The 3-*O*-sulfation increases the population of *2SO* conformer of the IdoA2S residue in hexasaccharides (compound **1** to **3,** Table 2 and FIG. 1A), compared that of the IdoA2S residue in a hexasaccharide without the GIcNS3S6S residue (compound **7).** In compound **3,** the IdoA2S residue almost exclusively displays the *2SO* conformation. For octasaccharides (compound **4** to **6,** Table 2 and FIG. 1A), two IdoA2S residues exist, designated as residue c and e. The effect of 3-*O*-sulfation on the *2SO* population varies. For example, 3-*O*-sulfation increases the *2SO* population for both flanked IdoA2S residues as displayed in compound **5,** compared with those of IdoA2S residues in an octasaccharide without the GIcNS3S6S residue (compound **10).** In compound **11,** the 3-*O*-sulfation on residue f increases *2SO* population of the adjacent IdoA2S residue (residue e), but has no effect on a distant IdoA2S residue (residue c), compared with corresponding residues in compound **10.** In compound **6,** 3-*O*-sulfation on residue d and f increases the *2SO* population of residue e, but decreases *2SO* population of residue c to 58% from 71% (Table 2), compared with the corresponding residues from compound **5.** These observations suggest that 3-*O*-sulfation could regulate the structure of HS through its effect on the conformation of IdoA2S residues.

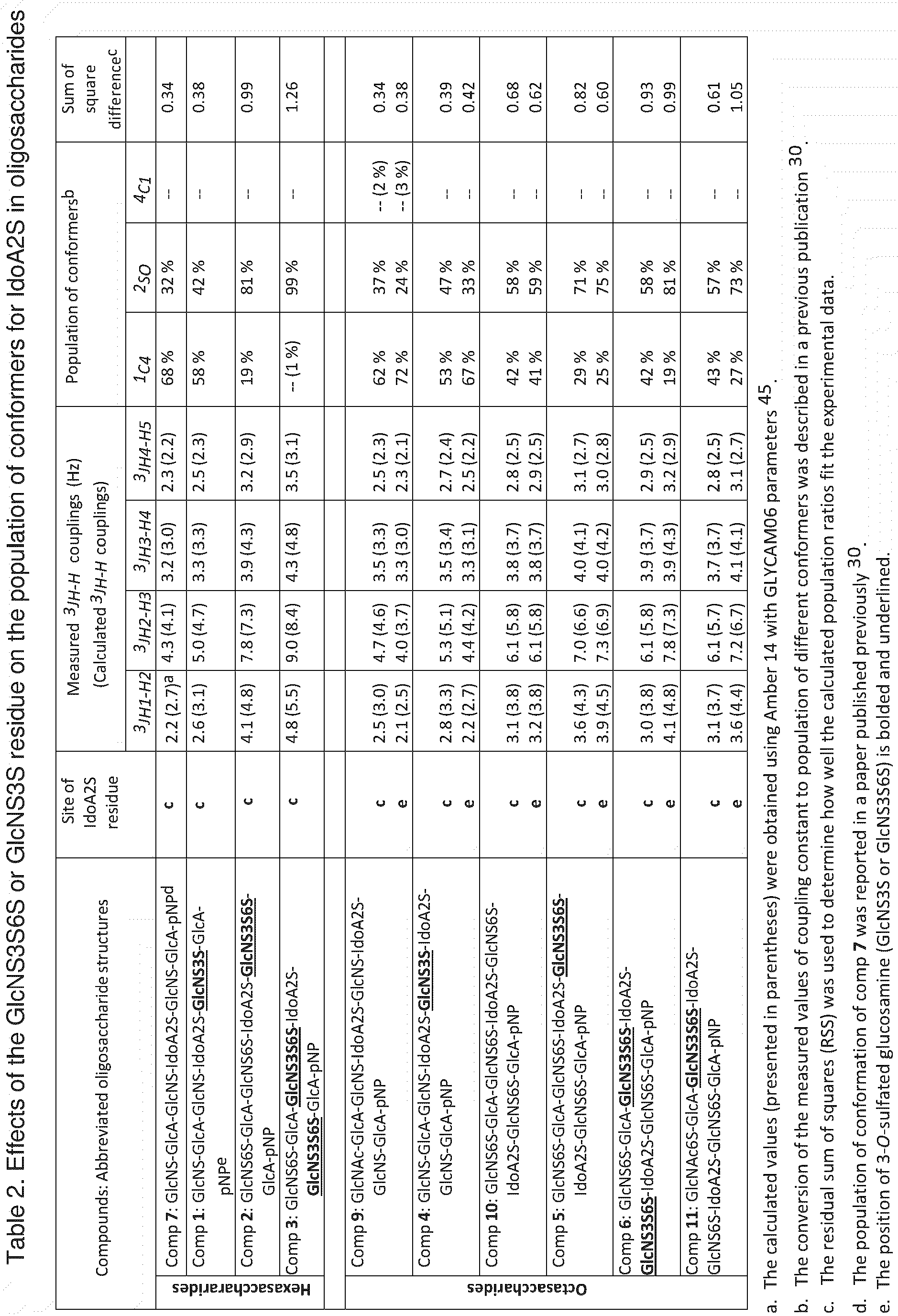

### EXAMPLE 3

### The presence of the -GIcNS3S6S-IdoA2S- disaccharide unit impacts anticoagulant activity.

HS oligosaccharides achieve their anticoagulant activity by interacting with AT, therefore oligosaccharides displaying anti-FXa activity should bind to AT. The effects of saccharide residues surrounding the GIcNS3S6S residue on the AT-binding as well as anti-FXa activity were examined. These data reveal that the presence of a -GlcNS3S6S-IdoA2S- disaccharide unit, *i.e.* an IdoA2S located at the reducing end of the GIcNS3S6S residue, can play a role in determining anticoagulant activity (Table 3). Compound **2** and **8** have six residues, eight sulfo groups, one IdoA2S and two GIcA residues, but only compound **8** displayed anti-FXa activity (FIG. 3A and Table 3). As expected, AT binds to compound **8** tightly with a Kd value of 7 ± 2 nM, but not to compound **2** (Table 3). Structurally, the compounds differ in the location of the IdoA2S residue. In compound **8,** the 3-O-sulfated glucosamine (GlcNS3S6S) residue is flanked by an IdoA2S residue at the reducing end such that it possesses the -GIcNS3S6S-IdoA2S- disaccharide unit, whereas the GIcNS3S6S in compound **2** is flanked by a GIcA residue at the reducing end such that it possesses the -GIcNS3S6S-GIcA- disaccharide unit.

**Table 3. Correlation between the disaccharide domain (-GlcNS3S6S-IdoA2S-) and AT-binding constant (Kd) as well as anti-FXa activity (IC50)**

| | **Abbreviated structures** | **Binding affinity (Kd) to AT (nM)** | **Anti-FXa activity (IC50, nM)** |
|---|---|---|---|
| Fondaparinux, 5-mer | GlcNS6S-GlcA-**GlcNS3S6S-IdoA2S**-GlcNS6S-OMe^{a} | 14.8 ± 1.4 nM | 12.2 nM |

| Hexasaccharides | | | |
|---|---|---|---|
| Comp **8**^{b} | GlcNS6S-GlcA-**GlcNS3S6S-IdoA2S**-GlcNS6S-GlcA-pNP | 7 ± 2 nM^{b} | 9.1 nM |
| Comp **2** | GlcNS6S-GlcA-GlcNS6S-IdoA2S-GlcNS3S6S-GlcA-pNP | No binding | No inhibition |
| Comp **3** | GlcNS6S-GlcA-**GlcNS3S6S-IdoA2S-**GlcNS3S6S-GlcA-pNP | Not measured | 11.0 nM |
| IdoA-containing 6-mer | GlcNS6S-GlcA-**GlcNS3S6S-IdoA-**GlcNS6S-GlcA-pNP^{c} | 32.6 ± 5.3 nM^{d} | 29.2 nM^{d} |
| GlcA2S- containing 6-mer | GlcNS6S-GlcA-GlcNS3S6S-GlcA2S-GlcNS6S-GlcA-pNP⁴ | No binding^{d} | Not measured |

| Octasaccharides | | | |
|---|---|---|---|
| Comp **4** | GlcNS-GlcA-GlcNS-IdoA2S-GlcNS3S-IdoA2S-GlcNS- GlcA-pNP | No binding^{c} | No inhibition |
| Comp **5** | GlcNS6S-GlcA-GlcNS6S-IdoA2S-GlcNS3S6S-IdoA2S- GlcNS6S-GlcA-pNP | 5.1 ± 1.4 nM | 7.7 nM |
| Comp **6** | GlcNS6S-GlcA-GlcNS3S6S-IdoA2S-GlcNS3S6S-IdoA2S- GlcNS6S-GlcA-pNP | 5.6 ± 2.6 nM | 10.9 nM |
| Comp **11** | GlcNAc6S-GlcA-GlcNS3S6S-IdoA2S-GlcNS6S-IdoA2S- GlcNS6S-GlcA-pNP | 8 ± 3 nM1 | 9.4 nM |

| | | | |
|---|---|---|---|
| a. Fondaparinux, an FDA-approved anticoagulant, was used as a positive control for anti-FXa activity measurement and the binding to AT. The -GlcNS3S6S-IdoA2S-disaccharide units are presented in bold face and underlined. b. The binding constants for comp **8** and comp **11** were determined by affinity coelectrophoresis as reported in a previous publication¹⁷. c. Comp **4** did not bind to AT nor display anti-FXa activity, due to the fact that it does not contain 6-*O-*sulfo groups at the GIcNS residues at the nonreducing end²⁹. d. The IdoA-containing 6-mer was reported in a previous publication ³⁰. IdoA also displays both *¹C4* and *²S_{O}* conformations. The GlcA2S-containing 6-mer was reported in a previous publication^{8.} | | | |

The findings herein are consistent with the previously published conclusion that the *²SO* conformation impacts anticoagulant activity⁹. The IdoA2S residue in compound **8** displays the *²SO* conformation; whereas in compound **2,** this position is occupied by a GIcA residue that adopts the *⁴C1* conformation. The X-ray crystal structure and NMR solution structure of the complex of AT and fondaparinux demonstrate that the IdoA2S residue is present in *²SO* conformation in the complex^{31,32}. An IdoA-containing 6-mer, where the IdoA2S residue was substituted with an IdoA residue displaying *²SO* conformation, exhibited anti-FXa activity³⁰ (Table 3). However, a GIcA2S-containing 6-mer, where the IdoA2S residue was substituted with a GIcA2S residue displaying *⁴C1* conformation, did not display anti-FXa activity⁸ (Table 2). Compound **3** displayed strong anti-FXa activity as the 6-mer consists of two GlcNS3S6S residues (FIG. 3A and Table 3), consistent with previously published results for an octasaccharide consisting a similar pentasaccharide domain³². Residues d and c in compound **3** constitute the -GIcNS3S6S-IdoA2S- disaccharide unit.

### EXAMPLE 4

### The GlcA residue in the AT-binding domain is substitutable by an IdoA2S residue

These studies uncovered a new AT-binding saccharide sequence. The currently known AT-binding sequence comprises a disaccharide -GIcA-GIcNS3S±6S- unit³³. The substitution of the -GlcA-GlcNS3S6S- disaccharide unit with the -IdoA2S-GIcNS3S6S- disaccharide unit in HS was perceived to abolish AT-binding affinity^{34,35}. The data from the anti-FXa activity and AT-binding analysis of the 8-mers clearly challenged this assertion. Compound **5,** which contains the -IdoA2S-GIcNS3S6S- (but not -GlcA-GlcNS3S6S-) disaccharide unit, displays strong anti-FXa activity (Table 3). The AT-binding affinity analysis using isothermal titration calorimetry (ITC) also demonstrated that compound **5** binds to AT tightly (FIG. 3B and Table 3). Compound **6** displays anti-FXa activity and high AT-binding affinity (Table 3), as this 8-mer contains the -GIcA-GIcNS3S6S- disaccharide unit.

The anticoagulant activity of compound **5** was further confirmed in an *in vivo* experiment using a rat model. To this end, compound **5** was administered and compared the anti-FXa effect with fondaparinux and compound **11,** an anticoagulant octasaccharide reported previously¹⁷. The results demonstrated that compound **5** has comparable anti-FXa potency to that of fondaparinux and compound **11** (FIG. 3C) within 30 min after the drug was administered. However, the anti-FXa effect from compound **5** diminished after 4 hours, while the anti-FXa effect from fondaparinux and compound **11** persisted after 8 hours (FIG. 3C). The drug concentrations in the blood sample were also obtained to determine the clearance rate of each compound *in vivo* (FIG. 3D). In comparison with fondaparinux and compound **11,** compound **5** was cleared faster from the animal in the first 2 hours.

The structural promiscuity for residue d in the AT-binding site (FIG. 4A) is supported by molecular dynamics (MD) simulations of AT in complex with variously modified HS pentasaccharides. The computational technique was first validated by substituting moieties that are known to be essential for AT-binding within an existing co-crystal structure of AT and fondaparinux^{24,36}. Calculations for the free energy of binding confirmed that removal of the 3-O-sulfate on residue c or substitution of residue b with GIcA destabilized the complex, indicated by significantly weakened interaction energy by 29% and 17%, respectively, qualitatively consistent with experimental data. In contrast, substitution of GIcA (residue d) with an IdoA2S in *¹C4* conformation had no effect on free energy (FIG. 4B). Interestingly, substitution with an IdoA2S residue in *²SO* conformation modestly enhanced the binding energy by 12% (FIG. 4B), suggesting the possibility of a more stable AT/pentasaccharide complex if residue d is IdoA2S in *²SO* conformer. IdoA2S in both conformations were capable of maintaining a similar position of the carboxyl moiety with respect to the protein as was found for the GIcA residue in the simulation of fondaparinux. Collectively, the MD data support the conclusion that substitution of residue d with an IdoA2S residue does not diminish the binding affinity to AT, and explain how Compound **5** acts as an active anticoagulant despite lacking the canonical pentasaccharide sequence for AT.

### EXAMPLE 5

### Analysis of 7-mer HS compound

A 7-mer HS compound or heparin analogue was developed using the disclosed synthesis methods. The 7-mer, the structure of which is shown in FIG. 8B, was tested for and demonstrates anti-Xa activity (FIG. 8A). FIG. 8B schematically compares the chemical structural of the 7-mer to a 6-mer and 8-mer as disclosed herein. By way of example and not limitation, at least one synthetic pathway for synthesizing the 7-mer is shown in FIG. 7C. In some embodiments, in can be preferable to have a relatively shorter oligosaccharide, such as a 7-mer, since this can allow for a reduction in costs associated with synthesis.

### EXAMPLE 6

### Discussion of Results

Described herein are schemes using the chemoenzymatic approach to prepare a 3-O-sulfated oligosaccharide library. It is demonstrated herein that the 3-OST-3 modification, to synthesize oligosaccharides comprising the - IdoA2S-GIcNS3S- or -IdoA2S-GIcNS3S6S- disaccharide unit, must precede the 6-O-sulfation step, whereas 3-OST-1 modification can occur only after 6-O-sulfation in order to generate the -GlcA-GlcNS3S6S- disaccharide unit. This finding is supported by the ternary cocrystal structures of 3-OST-1/heptasaccharide/PAP³⁷ and 3-OST-3/tetrasaccharide/PAP³⁸. There were no interactions between 3-OST-3 and the 6-*O*-sulfo groups from the tetrasaccharide substrate, consistent with the conclusion that oligosaccharide substrates for 3-OST-3 do not require 6-*O*-sulfation. In contrast, interactions between 3-OST-1 and the 6-*O*-sulfo groups from the heptasaccharide substrate were observed, suggesting that 6-*O*-sulfation is required to bind to 3-OST-1. The distinct and unique substrate requirements between 3-OST-1 and 3-OST-3 raise a possibility that 3-*O*-sulfated HS modified by different isoforms of 3-OST are biosynthesized through different pathways.

It was widely accepted that the 3-OST-1 enzyme is responsible for synthesizing anticoagulant HS, whereas the 3-OST-3 enzyme is not^{34,35}. The AT-binding sequences isolated so far all consist of the -GIcA-GIcNS3S6S-disaccharide repeating unit, which is a product of 3-OST-1 enzyme modification^{33,39}. Although Compound **5,** which is a product of 3-OST-3 enzyme modification, does not contain the -GIcA-GIcNS3S6S- disaccharide unit, it binds to AT and displays anticoagulant activity. These findings suggest that 3-OST-3 is capable of synthesizing anticoagulant HS, as long as the HS comprises the structural domain similar to that of compound **5.**

The fast clearance of compound **5** offers a potential new short-acting anticoagulant drug candidate with reduced bleeding risk. A short-acting anticoagulant drug, which can be cleared from the circulation quickly before major bleeding effects developed, would be particularly beneficial to those patients with high bleeding risk⁴¹. Although unfractionated heparin is an anticoagulant with a short half-life, the concern is that the drug causes heparin-induced thrombocytopenia (HIT), a life-threatening side effect⁴². It has been found that short oligosaccharides smaller than 12-mers⁴³ do not bind to platelet factor 4, and thus display no risk of HIT. As an octasaccharide, compound **5** is hence expected to have very low risk of HIT.
The availability of 3-O-sulfated oligosaccharides also opens the opportunity to investigate the casual relationship between saccharide sulfation/conformation and biological functions, a major step forward in dissecting the structure and function relationship of HS. HS 3-OST exists in seven different isoforms. In the present study, we demonstrate that different chemoenzymatic schemes are required for 3-OST-1 and 3-OST-3 to prepare different 3-*O*-sulfated oligosaccharide sequences. The conclusion from this study will guide others to develop the chemoenzymatic method using different 3-OST isoforms, allowing the preparation of more complex HS saccharides with more extensive sulfation patterns involving 3-*O*-sulfated glucosamine residues. These studies will enrich the HS oligosaccharide library to assist HS-related research.

### REFERENCES

(1) Xu, D.; Esko, J. Annu Rev Biochem. 2014, 83, 129.
(2) Xu, D.; Olson, J.; Cole, J. N.; van Wijk, X. M.; Brinkmann, V.; Zychlinsky, A.; Nizet, V.; Esko, J. D.; Chang, Y. C. Infect. Immun. 2015, 83, 3648.
(3) Patel, V. N.; Lombaert, I. M. A.; Cowherd, S. N.; Shworak, N.; Xu, Y.; Liu, J.; Hoffman, M. P. Developmental cell 2014, 29, 662.
(4) Liu, J.; Linhardt , R. J. Nat Prod Rep 2014, 31, 1676.
(5) Kreuger, J.; Spillmann, D.; Li, J.-p.; Lindahl, U. J. Cell Biol. 2006, 174, 323.
(6) Sattelle, B. M.; Hansen, S. U.; Gardiner, J. M.; Almond, A. J Am Chem Soc 2010, 132, 13132.
(7) Sattelle, B. M.; Almond, A. Glycobiology 2011, 21, 1651.
(8) Hsieh, P.; Xu, Y.; Keire, D. A.; Liu, J. Glycobiology 2014, 24, 681.
(9) Das, S.; Mallet, J.; J, E.; Driguez, P.; Duchaussoy, P.; Sizun, P.; Herault, J.; Herbert, J.; M, P.; P, S. Chemistry 2001, 7, 4821.
(10) Raman, R.; Venkataraman, G.; Ernst, S.; Sasisekharan, R. Proc. Natl. Acad. Sci. 2003, 100, 2357*.*
(11) Hu, Y.-P.; Lin, S.-Y.; Huang, C.-Y.; Zulueta, M. M. L.; Liu, J.-Y.; Chang, W.; Hung, S.-C. Nat Chem 2011, 3, 557.
(12) Arungundram, S.; Al-Mafraji, K.; Asong, J.; Leach III, F. E.; Amster, I. J.; Venot, A.; JE, T.; Boons, G. J. J. Am. Chem. Soc. 2009, 131, 17394.
(13) Schworer, R.; Zubkova, O. V.; Turnbull, J. E.; Tyler, P. C. Chem. Eur. J. 2013, 19, 6817.
(14) Zong, C.; Huang, R.; Condac, E.; Chiu, Y.; Xiao, W.; Li, Z. Q.; Lu, W.; Ishihara, M.; Wang, S.; Ramiah, A.; Stickney, M.; Azadi, P.; Amster, I. J.; Moremen, K. W.; Wang, L.; Sharp, J. S.; Boons, G.-J. J. Am. Chem. Soc. 2016, 138, 13059.
(15) Hansen, S. U.; Miller, G. J.; Cole, C.; Rushton, G.; Avizienyte, E.; Jayson, G. C.; Gardiner, J. M. Nat Commun 2013, 4:2016, doi: 10.1038/ncomms3016.
(16) Xu, Y.; Masuko, S.; Takieddin, M.; Xu, H.; Liu, R.; Jing, J.; Mousa, S. A.; Linhardt , R. J.; Liu, J. Science 2011, 334, 498*.*
(17) Xu, Y.; Cai, C.; Chandarajoti, K.; Hsieh, P.; Lin, Y.; Pham, T. Q.; Sparkenbaugh, E. M.; Sheng, J.; Key, N. S.; Pawlinski, R. L.; Harris, E. N.; Linhardt , R. J.; Liu, J. Nat Chem Biol2014, 10, 248*.*
(18) Thacker, B. E.; Seamen, E.; Lawrence, R.; Parker, M. W.; Xu, Y.; Liu, J.; Vander, K. C. W.; Esko, J. D. ACS Chem. Biol. 2016, 11, 971.
(19) Lindahl, U.; Backstrom, G.; Thunberg, L.; Leder, I. G. Proc. Natl. Acad. Sci. 1980, 77, 6551.
(20) Shukla, D.; Liu, J.; Blaiklock, P.; Shworak, N. W.; Bai, X.; Esko, J. D.; Cohen, G. H.; Eisenberg, R. J.; Rosenberg, R. D.; Spear, P. G. Cell 1999, 99, 13.
(21) Kamimura, K.; Rhodes, J. M.; Ueda, R.; McNeely, M.; Shukla, D.; Kimata, K.; Spear, P. G.; Shworak, N. W.; Nakato, H. J. Cell Biol. 2004, 166, 1069.
(22) Tecle, E.; Diaz-Balzac, C. A.; Bulow, H. E. G3 (Bethesda) 2013, 3, 541.
(23) Tsau, C.; Ito, M.; Gromova, A.; Hoffman, M. P.; Meech, R.; Makarenkova, H. P. Development 2011, 138, 3307*.*
(24) Petitou, M.; van Boeckel, C. A. A. Angew. Chem. Int. Ed. 2004, 43, 3118.
(25) Liu, J.; Shriver, Z.; Pope, R. M.; Thorp, S. C.; Duncan, M. B.; Copeland, R. J.; Raska, C. S.; Yoshida, K.; Eisenberg, R. J.; Cohen, G.; Linhardt, R. J.; Sasisekharan, R. J. Biol. Chem. 2002, 277, 33456.
(26) Liu, J.; Pedersen, L. C. Appl. Microbiol. Biotechnol. 2007, 74, 263.
(27) Chen, J.; Avci, F. Y.; Muñoz, E. M.; McDowell, L. M.; Chen, M.; Pedersen, L. C.; Zhang, L.; Linhardt , R. J.; Liu, J. J. Biol. Chem. 2005, 280, 42817.
(28) Zhang, Z.; McCallum, S. A.; Xie, J.; Nieto, L.; Corzana, F.; Jiménez-Barbero, J.; Chen, M.; Liu, J.; Linhardt, R. J. J Am Chem Soc 2008, 130, 12998.
(29) Atha, D. H.; Lormeau, J.-C.; Petitou, M.; Rosenberg, R. D.; Choay, J. Biochemistry 1985, 24, 6723.
(30) Hsieh, P.-H.; Thieker, D. F.; Guerrini, M.; Woods, R. J.; Liu, J. Sci Rep 2016, 6, 29602; doi: 10.1038/srep29602.
(31) Jin, L.; Abrahams, P.; Skinner, R.; Petitou, M.; Pike, R. N.; Carrell, R. W. Proc. Natl. Acad. Sci. 1997, 94, 14683.
(32) Guerrini, M.; Elli, S.; Mourier, P.; Rudd, T. R.; Gaudesi, D.; Casu, B.; Boudier, C.; Torri, G.; Viskov, C. Biochem. J. 2013, 449, 343.
(33) Guerrini, M.; Mourier, P. A.; Torri, G.; Viskov, C. Glycoconj. J. 2014, 31, 409.
(34) Liu, J.; Shworak, N. W.; Sinaÿ, P.; Schwartz, J. J.; Zhang, L.; Fritze, L. M. S.; Rosenberg, R. D. J. Biol. Chem. 1999, 274, 5185.
(35) Xu, D.; Moon, A.; Song, D.; Pedersen, L. C.; Liu, J. Nat Chem Biol 2008, 4, 200.
(36) Langdown, J.; Belzar, K. J.; Savory, W. J.; Baglin, T. P.; Huntington, J. A. J. Mol. Biol. 2009, 386, 1278.
(37) Moon, A. F.; Xu, Y.; Woody, S.; Krahn, J. M.; Linhardt , R. J.; Liu, J.; Pedersen, L. C. Proc. Natl. Acad. Sci. USA 2012, 109, 5256*.*
(38) Moon, A.; Edavettal, S. C.; Krahn, J. X.; Munoz, E. M.; Negishi, M.; Linhardt, R. J.; Liu, J.; Pedersen, L. C. J. Biol. Chem. 2004, 279, 45185.
(39) deAgostini, A. I.; Dong, J.-C.; de Vantery Arrighi, C.; Ramus, M.-A.; Dentand-Quadri, I.; Thanlmann, S.; Ventura, P.; Ibecheole, V.; Monge, F.; Fischer, A.-M.; HajMohammadi, S.; Shworak, N.; Zhang, L.; Zhang, Z.; Linhardt , R. J. J. Biol. Chem. 2008, 283, 28115.
(40) HajMohammadi, S.; Enjyoji, K.; Princivalle, M.; Christi, P.; Lech, M.; Beeler, D. L.; Rayburn, H.; Schwartz, J. J.; Barzegar, S.; de Agostini, A. I.; Post, M. J.; Rosenberg, R. D.; Shworak, N. W. J. Clin. Invest. 2003, 111, 989.
(41) Mahe, I.; Chidac, J.; Helfer, H.; Nobel, S. J. Thromb. Haemost. 2016, 14, 2017.
(42) McGowan, K. E.; Makari, J.; Diamantouros, A.; Bucci, C.; Rempel, P.; Selby, R.; Geerts, W. Blood 2016, 127, 1954.
(43) Kreimann, M.; Brandt, S.; Krauel, K.; Block, S.; Helm, C.; Weitschies, W.; Greinacher, A.; Delcea, M. Blood 2014, in press.
(44) Yang, J.; Hsieh, P.; Liu, X.; Zhou, W.; Zhang, X.; Zhao, J.; Xu, Y.; Zhang, F.; Linhardt, R. J.; Liu, J. Chem Comm 2017, 53, 1743.
(45) Kirschner, K. N.; Yongye, A. B.; Tschampel, S. M.; González-Outeiriño, J.; Daniels, C. R.; Foley, B. L.; Woods, R. J. J. Comput. Chem. 2008, 29, 622.
(46) Xu, Y.; Cai, C.; Chandarajoti, K.; Hsieh, P.; Lin, Y.; Pham, T. Q.; Sparkenbaugh, E. M.; Sheng, J.; Key, N. S.; Pawlinski, R. L.; Harris, E. N.; Linhardt , R. J.; Liu, J. Nat Chem Biol 2014, 10, 248*.*
(47) Langdown, J.; Belzar, K. J.; Savory, W. J.; Baglin, T. P.; Huntington, J. A. J. Mol. Biol. 2009, 386, 1278.
(48) Pettersen, E. F.; Goddard, T. D.; Huang, C. C.; Couch, G. S.; Greenblatt, D. M.; Meng, E. C.; Ferrin, T. E. J. Comp. Chem. 2004, 25, 1605.
(49) Fiser, A.; Sali, A. Methods Enzymol 2003, 374, 461.
(50) Huang, C. C.; Meng, E. C.; Morris, J. H.; Pettersen, E. F.; Ferrin, T. E. Nucleic Acids Res. 2014, 42, w478.
(51) Yang, Z.; Lasker, K.; Schneidman-Duhovny, D.; Webb, B.; Huang, C. C.; Pettersen, E. F.; Goddard, T. D.; Meng, E. C.; Sali, A.; Ferrin, T. E. J. Struct. Biol. 2012, 179, 269*.*
(52) Case, D. A.; Darden, T. A.; Cheatham III, T. E.; Simmerling, C. L.; Wang, J.; Duke, R. E.; Luo, R.; Walker, R. C.; Zhang, W.; Merz, K. M.; Roberts, B.; Hayik, S.; Roitberg, A.; Seabra, G.; Swails, J.; Goetz, A. W.; Kolossváry, I.; Wong, K. F.; Paesani, F.; Vanicek, J.; Wolf, R. M.; Liu, J.; Wu, X.; Brozell, S. R.; Steinbrecher, T.; Gohlke, H.; Cai, Q.; Ye, X.; Wang, J.; Hsieh, M.-J.; Cui, G.; Roe, D. R.; Mathews, D. H.; Seetin, M. G.; Salomon-Ferrer, R.; Sagui, C.; Babin, V.; Luchko, T.; Gusarov, S.; Kovalenko, A.; Kollman, P. A. **2014,** University of California.
(53) Kirschner, K. N.; Yongye, A. B.; Tschampel, S. M.; González-Outeiriño, J.; Daniels, C. R.; Foley, B. L.; Woods, R. J. J. Comput. Chem. 2008, 29, 622.
(54) Singh, A.; Tessier, M. B.; Pederson, K.; Wang, X.; Venot, A. P.; Boons, G.-J.; Prestegard, J. H.; Woods, R. J. Can. J. Chem. 2016, 10.1139/cjc.
(55) Hsieh, P.-H.; Thieker, D. F.; Guerrini, M.; Woods, R. J.; Liu, J. Sci Rep 2016, 6, 29602; doi: 10.1038/srep29602.
(56) Darden, T.; York, D.; Pedersen, L. C. J. Chem. Phys. 1993, 98, 10089.
(57) Kollman, P. A.; Massova, I.; Reyes, C.; Kuhn, B.; Huo, S.; Chong, L.; Lee, M.; Lee, T.; Duan, Y.; Wang, W.; Donini, O.; Cieplak, P.; Srinivasan, J.; Case, D. A.; Cheatham, T. E. r. Acc. Chem. Res. 2000, 33, 889.
(58) Onufriev, A.; Bashford, D.; Case, D. A. Proteins 2004, 55, 383.
(59) Humphrey, W.; Dalke, A.; Schulten, K. J. Mol. Graph. 1996, 14, 33.

## Claims

1. A synthetic heparin analogue for use in a method of treating a subject in need of anticoagulant therapy, wherein the synthetic heparin analogue comprises a structure selected from the group consisting of: 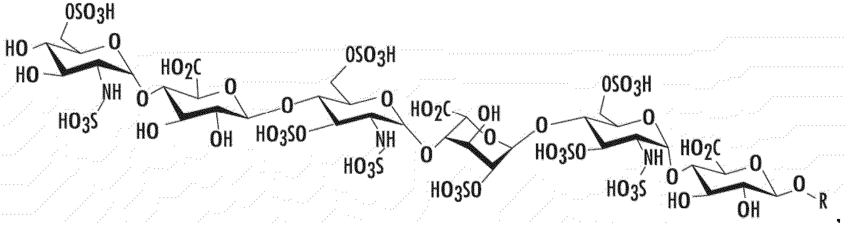 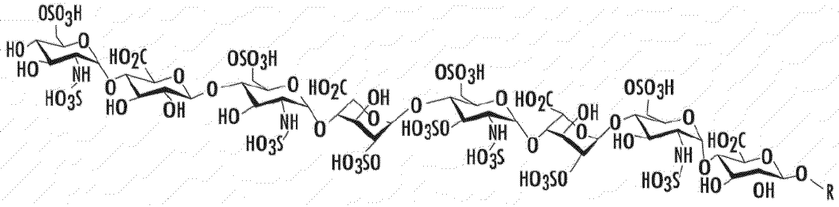 and 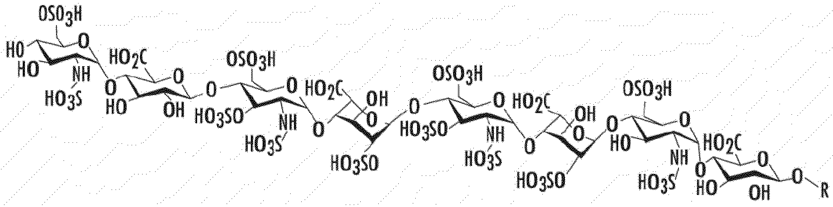 wherein R is selected from the group consisting of -H, alkyl (such as but not limited to -CH₃ or -CH₂CH₃), substituted alkyl, aryl, and substituted aryl (such as but not limited to a p-nitrophenyl group); or 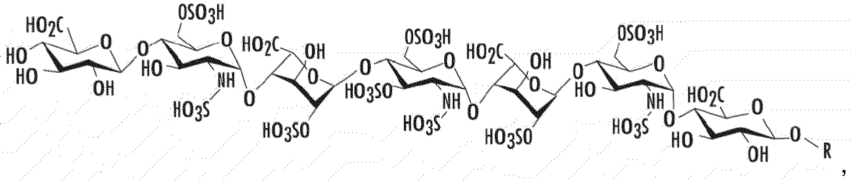 wherein R is selected from the group consisting of -H, alkyl (such as but not limited to -CH₃ or -CH₂CH₃), substituted alkyl, aryl, and substituted aryl (such as but not limited to a p-nitrophenyl group).

2. The synthetic heparin analogue for use of claim 1, wherein the synthetic heparin analogue comprises a structure selected from the group consisting of: 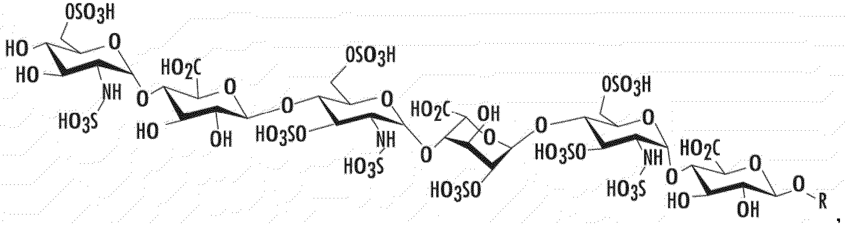 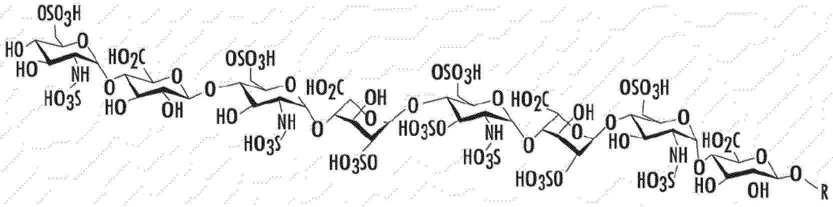 and 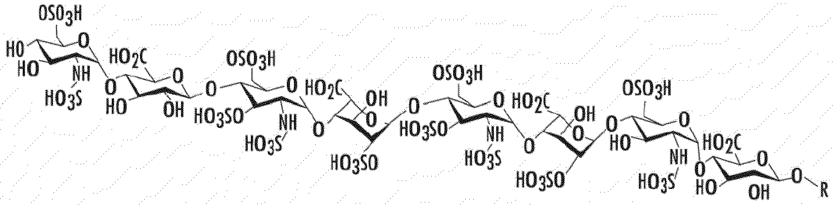 wherein R is selected from the group consisting of -H, alkyl (such as but not limited to -CH₃ or -CH₂CH₃), substituted alkyl, aryl, and substituted aryl (such as but not limited to a p-nitrophenyl group).

3. The synthetic heparin analogue for use of claim 1 or 2, said use further comprising monitoring the subject for heparin-induced thrombocytopenia, and administering to the subject an antidote to reverse the anticoagulant activity of the synthetic heparin analogue if the subject suffers from heparin-induced thrombocytopenia, optionally
wherein the antidote to reverse the anticoagulant activity of the synthetic heparin analogue is andexanet alfa, and/or
wherein the subject is a human subject, and/or wherein the synthetic heparin analogue has a clearance rate about 50% to about 100% faster than that of other heparin compounds, and/or
wherein the synthetic heparin analogue comprises an anticoagulant activity of less than about 10% at 4 hours post-administration, and/or
wherein the subject has an elevated risk of bleeding.

4. A pharmaceutical composition comprising a synthetic heparin analogue comprising a structure selected from 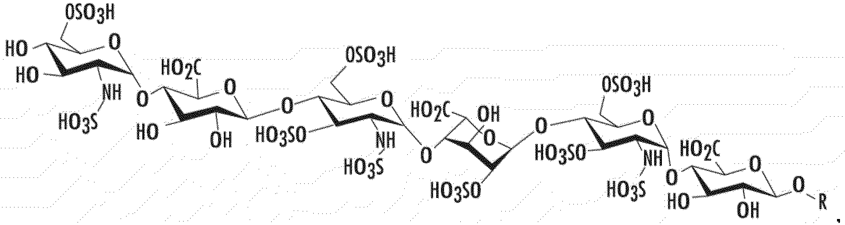 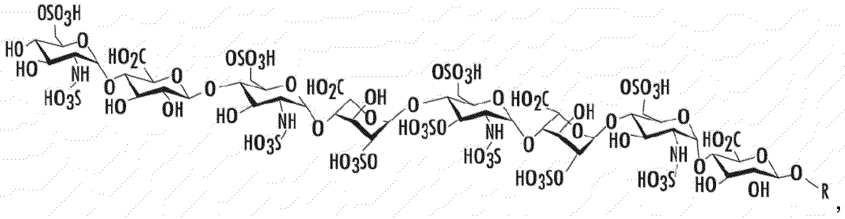 and 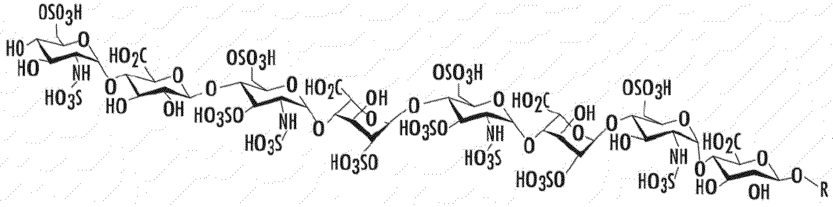 wherein R is selected from the group consisting of -H, alkyl (such as but not limited to -CH₃ or -CH₂CH₃), substituted alkyl, aryl, and substituted aryl (such as but not limited to a p-nitrophenyl group); or 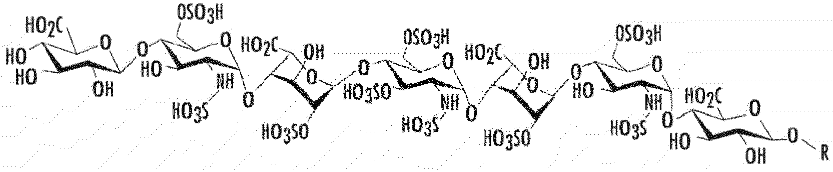 wherein R is selected from the group consisting of -H, alkyl (such as but not limited to -CH₃ or -CH₂CH₃), substituted alkyl, aryl, and substituted aryl (such as but not limited to a p-nitrophenyl group).

5. The pharmaceutical composition of claim 4, wherein the synthetic heparin analogue comprises a structure selected from the group consisting of: 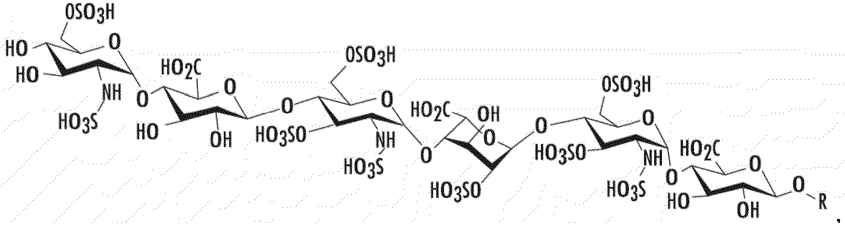 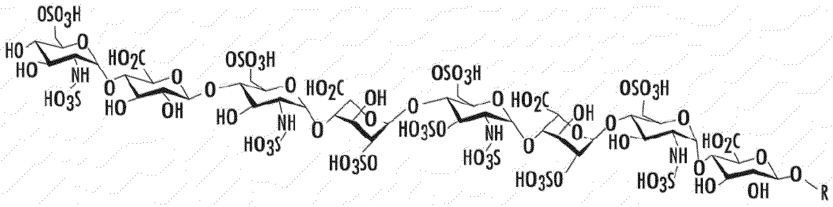 and 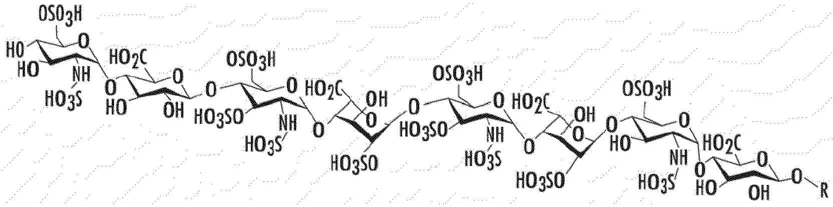 wherein R is selected from the group consisting of -H, alkyl (such as but not limited to -CH₃ or -CH₂CH₃), substituted alkyl, aryl, and substituted aryl (such as but not limited to a p-nitrophenyl group).

## Patentansprüche

1. Synthetisches Heparin-Analogon zur Verwendung in einem Verfahren zur Behandlung eines Individuums, das einer gerinnungshemmenden Therapie bedarf, wobei das synthetische Heparin-Analogon eine Struktur umfasst, die ausgewählt ist aus der Gruppe bestehend aus: 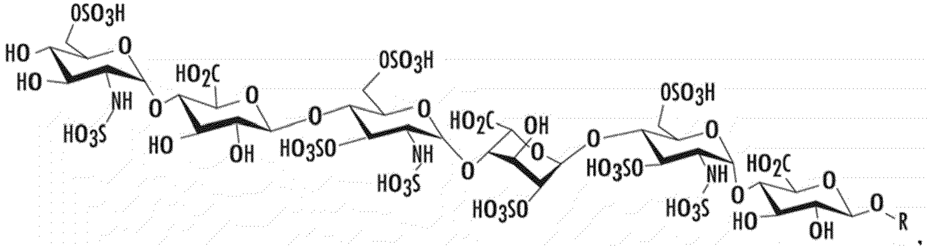 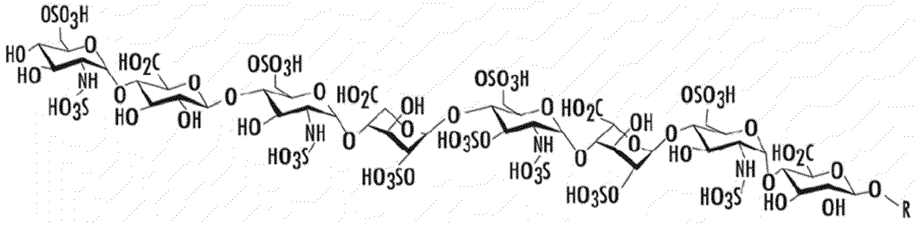 und 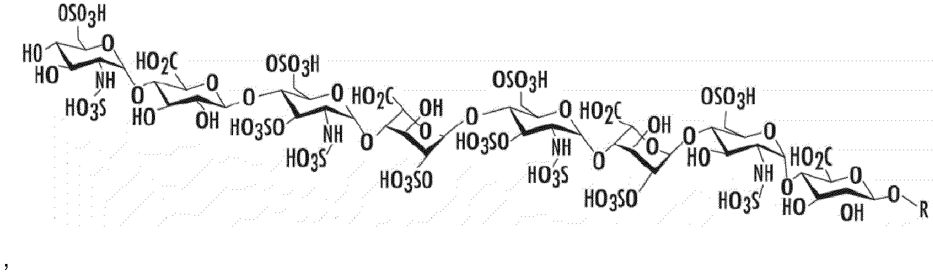 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe); oder 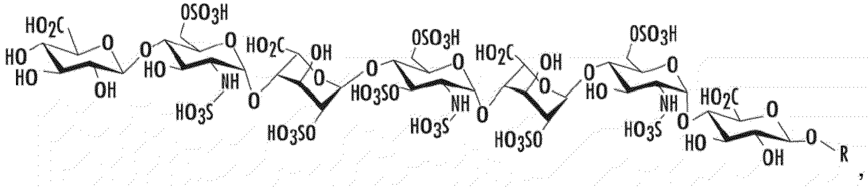 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe).

2. Synthetisches Heparin-Analogon zur Verwendung nach Anspruch 1, wobei das synthetische Heparin-Analogon eine Struktur umfasst, die ausgewählt ist aus der Gruppe bestehend aus: 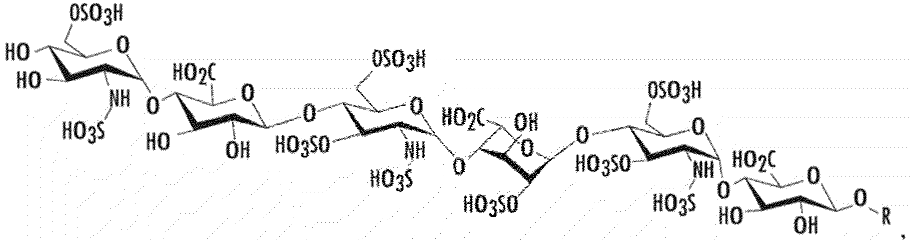 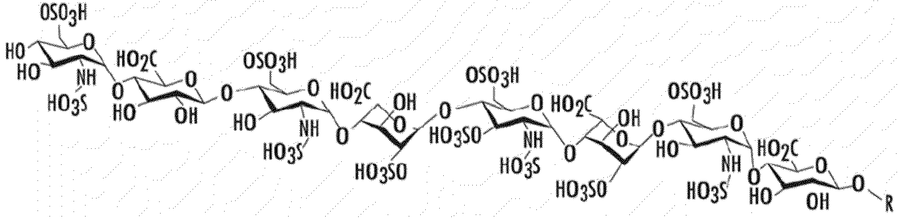 und 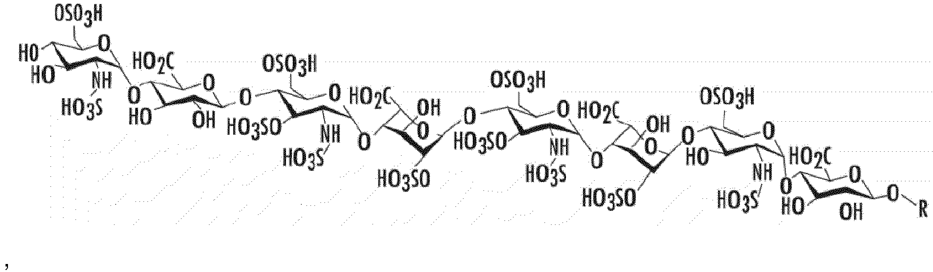 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe).

3. Synthetisches Heparin-Analogon zur Verwendung nach Anspruch 1 oder 2, wobei die Verwendung weiterhin die Überwachung des Individuums auf heparininduzierte Thrombozytopenie und die Verabreichung eines Antidots an das Individuum, um die gerinnungshemmende Wirkung des synthetischen Heparin-Analogons aufzuheben, falls das Individuum an heparininduzierter Thrombozytopenie leidet, umfasst, gegebenenfalls
wobei das Antidot zum Aufheben der gerinnungshemmenden Wirkung des synthetischen Heparin-Analogons Andexanet alfa ist, und/oder
wobei das Individuum ein menschliches Individuum ist, und/oder wobei das synthetische Heparin-Analogon eine Clearance-Rate etwa 50 % bis etwa 100 % schneller als die anderer Heparinverbindungen aufweist, und/oder
wobei das synthetische Heparin-Analogon 4 Stunden nach Verabreichung eine gerinnungshemmende Wirkung von weniger als etwa 10 % aufweist, und/oder
wobei das Individuum ein erhöhtes Blutungsrisiko aufweist.

4. Pharmazeutische Zusammensetzung, umfassend ein synthetisches Heparin-Analogon, das eine Struktur umfasst, die ausgewählt ist aus: 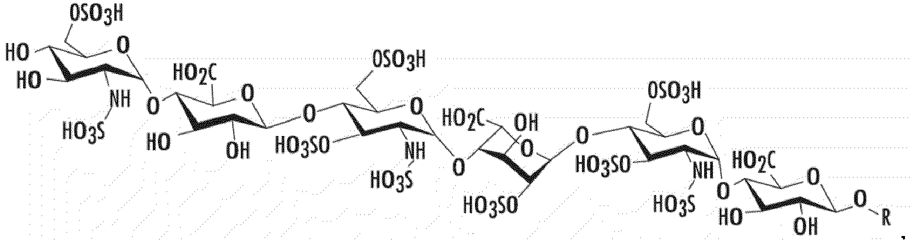 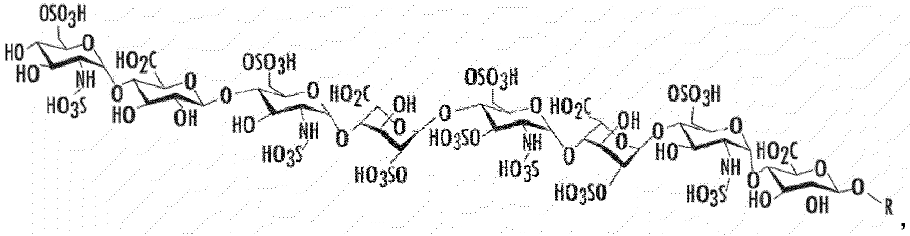 und 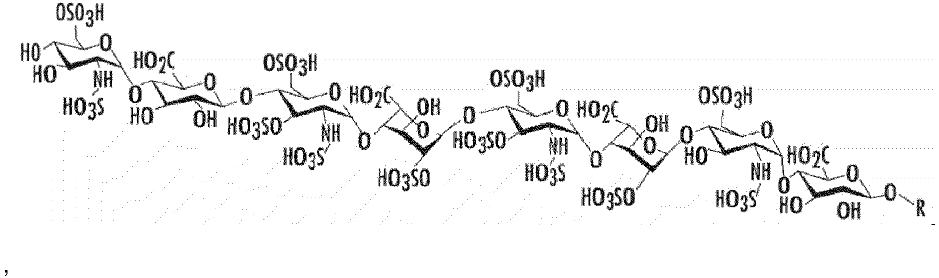 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe); oder 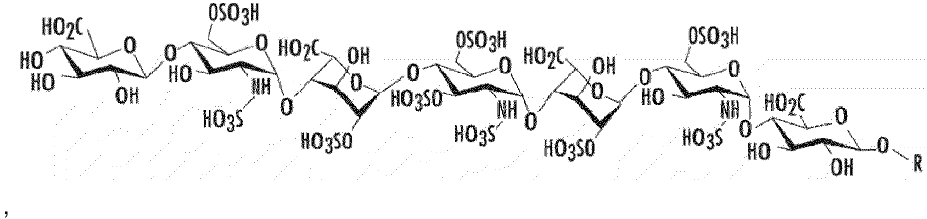 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe).

5. Pharmazeutische Zusammensetzung nach Anspruch 4, wobei das synthetische Heparin-Analogon eine Struktur umfasst, die ausgewählt ist aus der Gruppe bestehend aus: 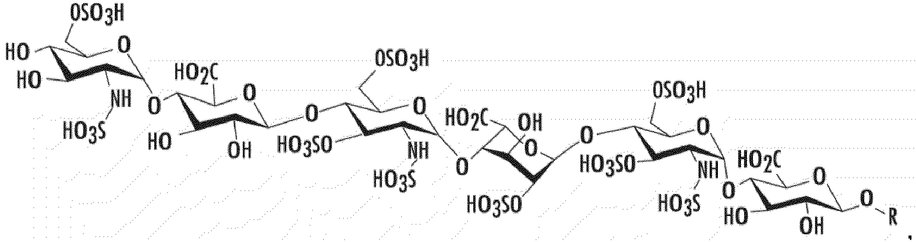 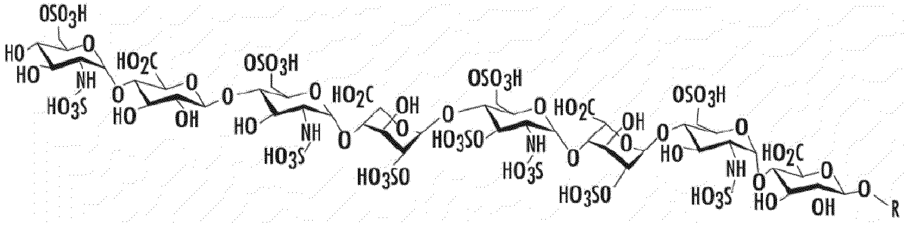 und 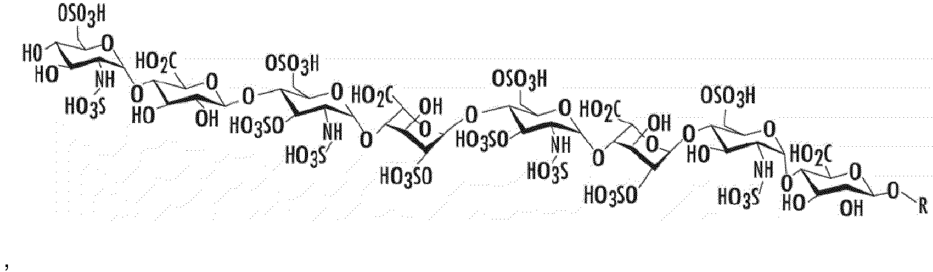 wobei R ausgewählt ist aus der Gruppe bestehend aus -H, Alkyl (wie zum Beispiel, aber nicht beschränkt auf, -CH₃ oder -CH₂CH₃), substituiertem Alkyl, Aryl und substituiertem Aryl (wie zum Beispiel, aber nicht beschränkt auf, eine *p*-Nitrophenylgruppe).

## Revendications

1. Analogue synthétique de l'héparine destiné à être utilisé dans un procédé de traitement d'un sujet nécessitant un traitement anticoagulant, dans lequel l'analogue synthétique de l'héparine comprend une structure choisie dans le groupe constitué par : 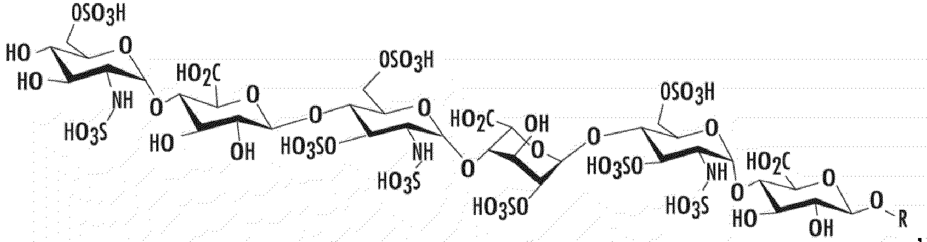 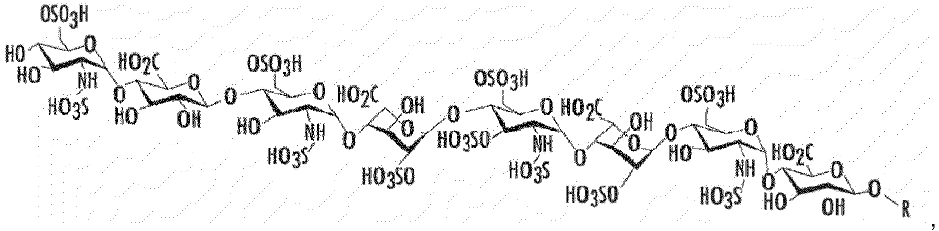 et 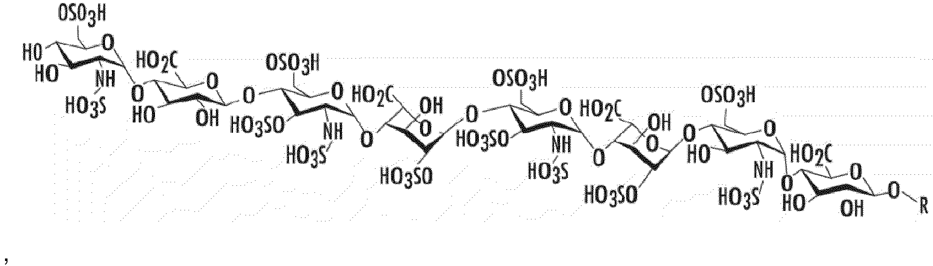 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe *p*-nitrophényle) ; ou 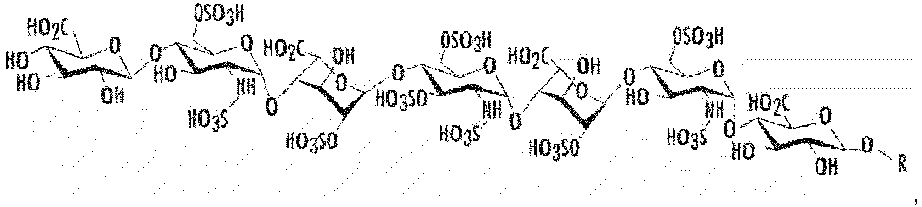 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe p-nitrophényle).

2. Analogue synthétique de l'héparine destiné à être utilisé selon la revendication 1, dans lequel l'analogue synthétique de l'héparine comprend une structure choisie dans le groupe constitué par : 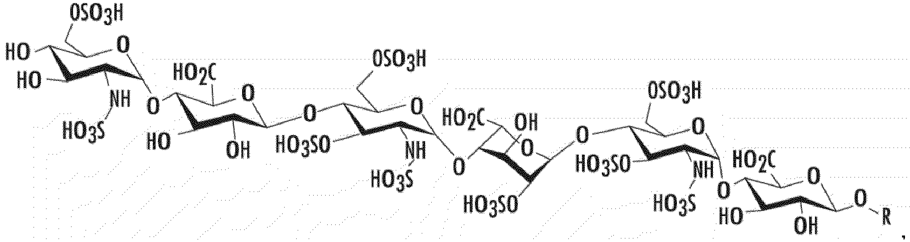 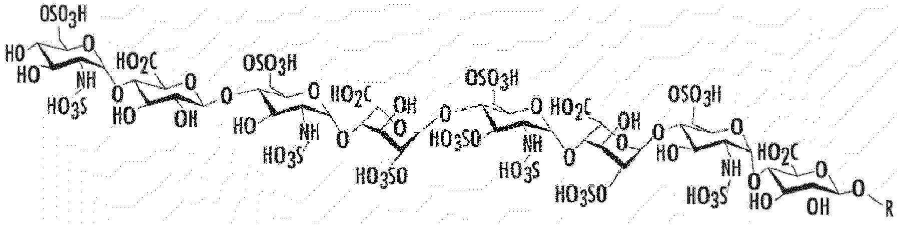 , et 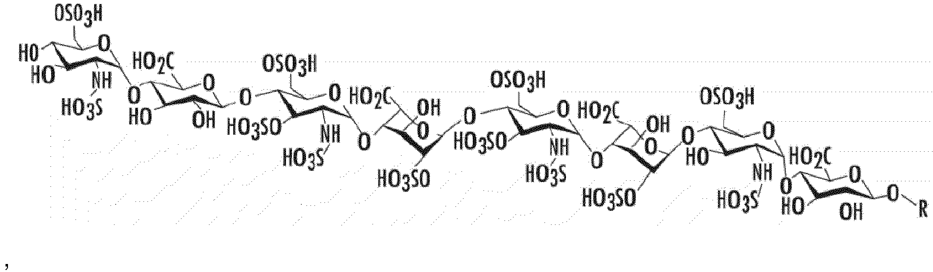 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe p-nitrophényle).

3. Analogue synthétique de l'héparine destiné à être utilisé selon la revendication 1 ou 2, ladite utilisation comprenant en outre la surveillance du sujet quant à l'apparition d'une thrombopénie induite par l'héparine et l'administration au sujet d'un antidote destiné à inverser l'activité anticoagulante de l'analogue synthétique de l'héparine si le sujet souffre d'une thrombopénie induite par l'héparine, facultativement
dans lequel l'antidote destiné à inverser l'activité anticoagulante de l'analogue synthétique de l'héparine est l'andexanet alfa, et/ou
dans lequel le sujet est un sujet humain, et/ou dans lequel l'analogue synthétique de l'héparine présente une vitesse d'élimination environ 50 % à environ 100 % supérieure à celle d'autres composés de l'héparine, et/ou
dans lequel l'analogue synthétique de l'héparine présente une activité anticoagulante inférieure à environ 10 % 4 heures après l'administration, et/ou
dans lequel le sujet présente un risque accru de saignement.

4. Composition pharmaceutique comprenant un analogue synthétique de l'héparine comprenant une structure choisie parmi : 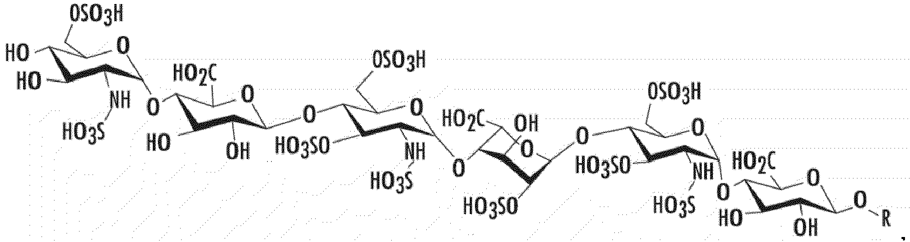 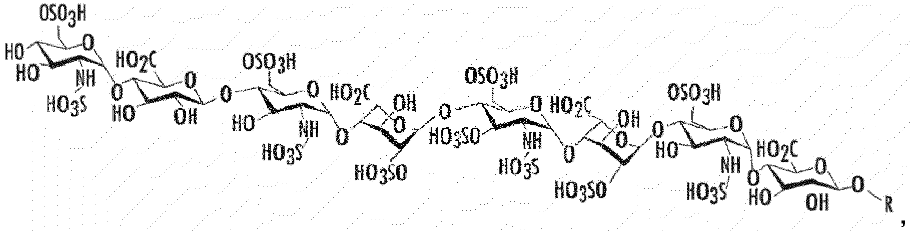 et 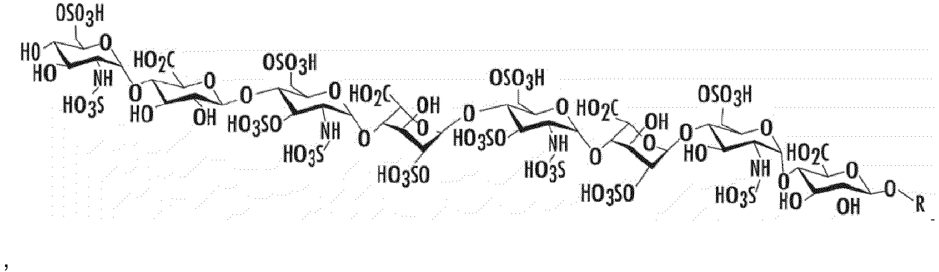 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe *p*-nitrophényle) ; ou 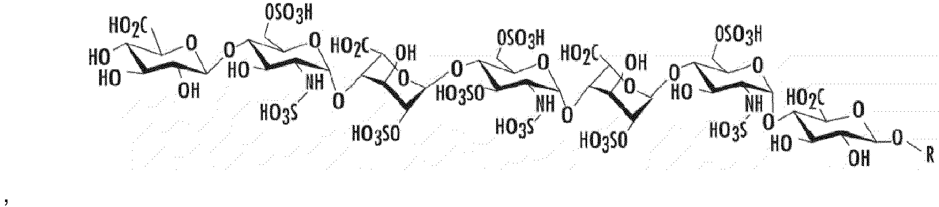 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe *p*-nitrophényle).

5. Composition pharmaceutique selon la revendication 4, dans laquelle l'analogue synthétique de l'héparine comprend une structure choisie dans le groupe constitué par : 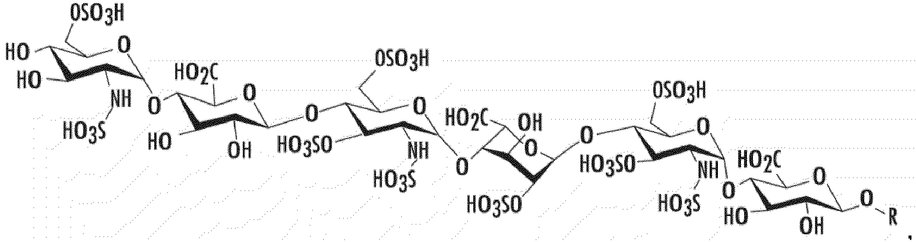 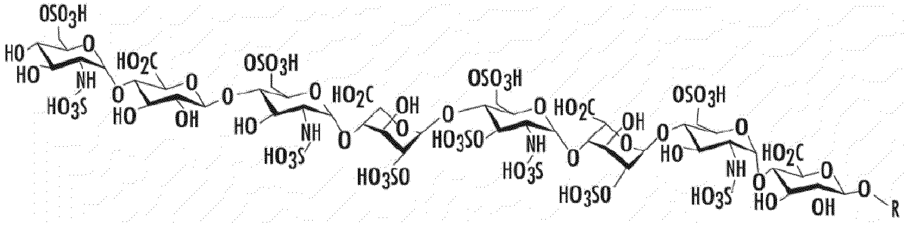 et 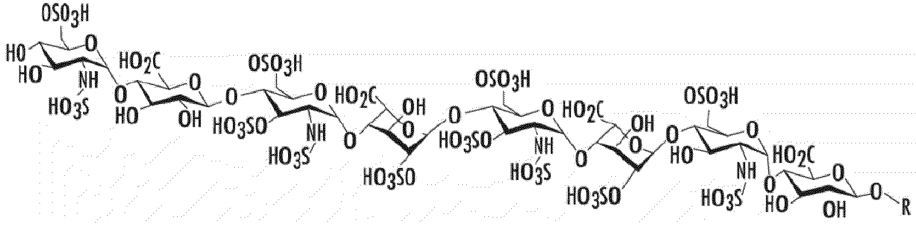 où R est choisi dans le groupe constitué par -H, un alkyle (tel que, sans toutefois s'y limiter, -CH₃ ou -CH₂CH₃), un alkyle substitué, un aryle, et un aryle substitué (tel que, sans toutefois s'y limiter, un groupe *p*-nitrophényle).
